# EUROPÄISCHE PATENTSCHRIFT

(11) **EP 0 649 454 B1**
(45) Veröffentlichungstag und Bekanntmachung des Hinweises auf die Patenterteilung: **16.12.1998**
(21) Anmeldenummer: 94915561.8
(22) Anmeldetag: 29.04.1994
(51) Int. Cl.: C09K 19/30, C07C 43/225, C09K 19/04

(54) **BENZOLDERIVATE UND FLÜSSIGKRISTALLINES MEDIUM**
BENZENE DERIVATIVES AND LIQUID-CRYSTAL MEDIUM
DERIVES DU BENZENE ET SUBSTANCE A CRISTAUX LIQUIDES

(30) Priorität: 10.05.1993 DE 4315410; 10.05.1993 DE 4315553; 10.05.1993 DE 4315555
(43) Veröffentlichungstag der Anmeldung: 26.04.1995
(73) Patentinhaber: MERCK PATENT GmbH, 64293 Darmstadt (DE)
(72) Erfinder: PLACH, Herbert, D-64287 Darmstadt (DE); PAULUTH, Detlef, D-64372 Ober-Ramstadt (DE); KRAUSE, Joachim, D-64807 Dieburg (DE); WEBER, Georg, D-64390 Erzhausen (DE); REIFFENRATH, Volker, D-64380 Rossdorf (DE); POETSCH, Eike, D-64367 Mühltal (DE)
(86) Internationale Anmeldenummer: EP9401364
(87) Internationale Veröffentlichungsnummer: WO9426838

(56) Entgegenhaltungen:
- EP-A- 0 507 094
- WO-A-91/08184
- WO-A-91/17135
- WO-A-92/13928
- DE-A- 4 111 991
- DE-A- 4 123 539
- DE-A- 4 142 519

## Beschreibung

Die vorliegende Erfindung betrifft Benzolderivate sowie ein flüssigkristallines Medium, dessen Verwendung für elektrooptische Zwecke und dieses Medium enthaltende Anzeigen.

Flüssige Kristalle werden vor allem als Dielektrika in Anzeigevorrichtungen verwendet, da die optischen Eigenschaften solcher Substanzen durch eine angelegte Spannung beeinflußt werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical Mode interference"). Die gebräuchlichsten Anzeigevorrichtungen beruhen auf dem Schadt-Helfrich-Effekt und besitzen eine verdrillt nematische Struktur.

Die Flüssigkristallnaterialien müssen eine gute chemische und thermische Stabilität und eine gute Stabilität gegenüber elektrischen Feldern und elektromagnetischer Strahlung besitzen. Ferner sollten die Flüssigkristallnaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem moglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, daß die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand guter UV- und Temperaturstabilität des Widerstands und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Große der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, daß je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfaßt hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobiloder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, daß der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, daß auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermoglichen:
- erweiterter nematischer Phasenbereich (insbesondere zu tiefen Temperaturen)
- Schaltbarkeit bei extrem tiefen Temperaturen (out-door-use, Automobil, Avionik)
- Erhöhte Beständigkeit gegenüber UV-Strahlung (längere Lebensdauer)

Mit den aus dem Stand der Technik zur Verfügung stehenden Medien ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klarpunkt, Übergang smektisch-nematisch bzw. Schmelzpunkt, Viskosität, dielektrische Größen, elastische Größen) dringend erwünscht.

Der Erfindung liegt die Aufgabe zugrunde, Medien insbesondere für derartige MFK-, TN- oder STN-Anzeigen bereitzustellen, die die oben angegebenen Nachteile nicht oder nur in geringerem Maße, und vorzugsweise gleichzeitig sehr hohe spezifische Widerstände und niedrige Schwellenspannungen aufweisen.

Es wurde nun gefunden, daß diese Aufgabe gelost werden kann, wenn man in Anzeigen erfindungsgemäße Medien verwendet.

Gegenstand der Erfindung ist somit ein flüssigkristallines Medium auf der Basis eines Gemisches von polaren Verbindungen mit positiver dielektrischer Anisotropie, dadurch gekennzeichnet, daß es eine oder mehrere Verbindungen der allgemeinen Formel I 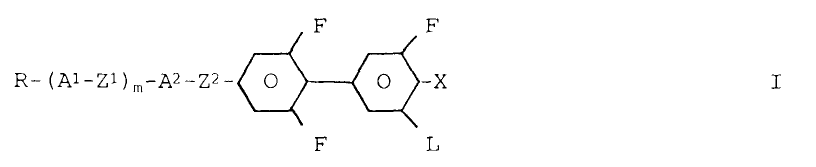 enthält,
zusätzlich eine oder mehrere Verbindungen ausgewählt aus der Gruppe bestehend aus den allgemeinen Formeln II, III, IV und V enthält: 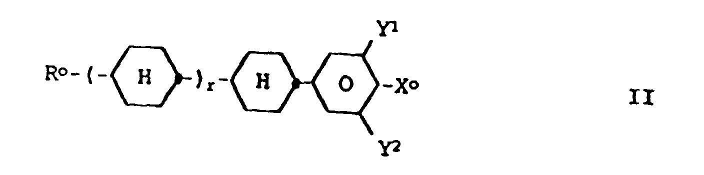 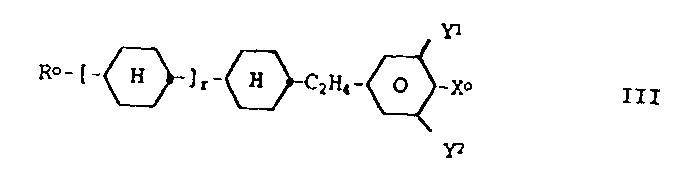 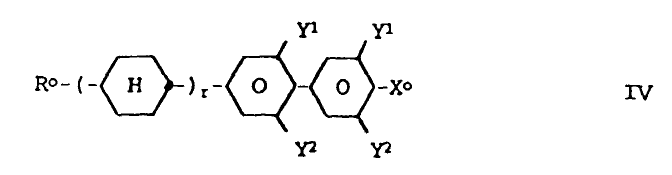 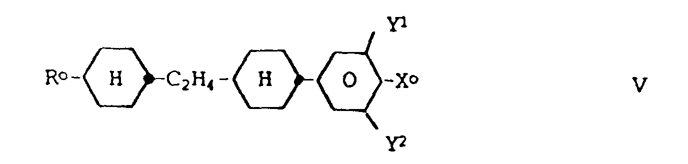 worin die einzelnen Reste die folgenden Bedeutungen haben:
- R: H, einen unsubstituierten, einen einfach durch CN oder CF₃ oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, -<>-, -CO-, -CO-O-, -O-CO- oder -O-CO-O- so ersetzt sein können, daß O-Atome nicht direkt miteinander verknüpft sind,
- A¹ und A²: jeweils unabhängig voneinander

(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch ein oder zwei Fluor substituiert sein können,
- Z¹ und Z²: jeweils unabhängig voneinander -CO-O-, -O-CO-, RH₂O-, -OCH₂-, -CH₂CH₂-, -CH=CH-, -C≡C- oder eine Einfachbindung, einer der Reste Z¹ und Z² auch -(CH₂)₄- oder -CH=CH-CH₂CH₂-,
- X: halogeniertes Alkyl, Alkoxy, Alkenyl oder Alkenyloxy mit jeweils 1 bis 6 C-Atomen,
- L: F und im Fall X = OCF₂H oder OC₂F₅ auch H, und
- m: 0, 1 oder 2,
- R^{o}:: Alkyl, Oxaalkyl, Fluoralkyl oder Alkenyl mit jeweils bis zu 7 C-Atomen,
- X^{o} :: F, Cl, CF₃, OZF₃ , OCHF₂ , CCH=CF₂, CCF=CF₂, CCFH=CF₂H oder OCF₂-CF₂H,
- Y¹ und Y²:: jeweils unabhängig voneinander H oder F,
- r:: 0 oder 1.

Aus der DE-A-4123533 ist ein sehr ähnliches Mischungskonzept bekannt. Die dort beschriebenen flüssigkristallinen Mischungen enthalten Dreiringverbindungen mit einer Trifluorbiphenyleinheit, wenn X Fluor oder OCF₃ bedeutet. Derartige Mischungen sind nicht Gegenstand der vorliegenden Erfindung.

Insbesondere bevorzugt sind Mischungen, die Verbindungen der Formel I sowie aller Teilformeln enthalten, in denen A¹ ein-oder zweifach durch F oder einfach durch CN substituiertes 1,4-Phenylen bedeutet. Insbesondere sind dies 2-Fluor- 1,4-phenylen, 3-Fluor-1,4-phenylen und 3,5-Difluor-1,4- phenylen sowie 2-Cyan-1,4-phenylen und 3-Cyan-1,4-phenylen.

In der Formel I bedeuten Z¹ und Z² bevorzugt eine Einfachbindung und -CH₂CH₂-, in zweiter Linie bevorzugt -CH₂O-, -OCH₂-, -O-CO-, und -CO-O-. Falls einer der Reste Z¹ und Z² -(CH₂)₄- oder -CH=CH-CH₂CH₂- bedeutet, so ist der andere Rest Z¹ oder Z² (falls vorhanden) vorzugsweise die Einfachbindung.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O-oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R einen einfach durch CN oder CF₃ substituierten Alkyl-oder Alkenylrest bedeutet, so ist dieser Rest vorzugsweise geradkettig. Die Substitution durch CN oder CF₃ ist in beliebiger Position.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I, die über für Polymerisationsreaktionen geeignete Flügelgruppen R verfügen, eignen sich zur Darstellung flüssigkristalliner Polymerer.

Verbindungen der Formel I mit verzweigten Flügel gruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)- heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis- (ethoxycarbonyl)-butyl, 5,5-Bis-(ethoxycarbonyl)-hexyl.

Die 1,4-Cyclohexenylen-Gruppe hat vorzugsweise folgende Strukturen: 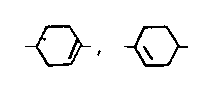 Gegenstand der Erfindung sind auch neue Verbindungen der Formeln I1 und I2: 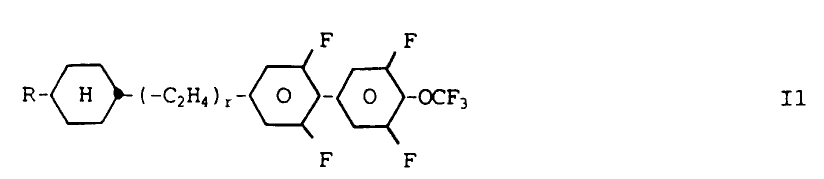 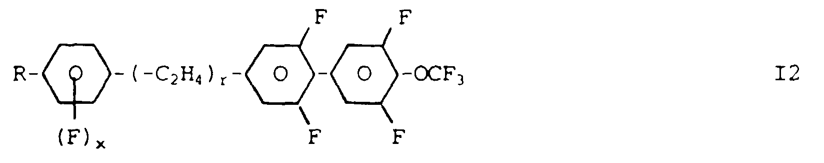 worin
R die oben angegebene Bedeutung hat und r 0 oder 1 und x 0, 1 oder 2 bedeuten.

Verbindungen der Formeln I1 und I2 werden zum Teil in den WO 89/02884, WO 91/03450 und WO 91/08184 durch allgemeine Formeln umfaßt. Es sind jedoch nicht die vorteilhaften Wirkungen der Tetrafluorbiphenyle als Komponenten von flüssigkristallinen Medien angegeben.

Die Verbindungen der Formeln I1 und I2 können, wie ähnliche, z.B. aus der WO 89/02884 bekannte Verbindungen als Komponenten flüssigkristalliner Medien verwendet werden, insbesondere für Anzeigen, die auf dem Prinzip der verdrillten Zelle beruhen. Sie besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Die bevorzugten Reste für Verbindungen der Formel I gelten auch für die Verbindungen I1 und I2.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Die erfindungsgemäßen Verbindungen können z.B. hergestellt, indem man eine Verbindung der Formel II*, 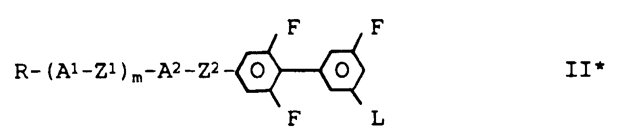 worin R, A¹, A², Z¹, Z², L und m die angegebene Bedeutung haben, gemäß folgendem Reaktionsschema metalliert und anschließend mit einem geeigneten Elektrophil umsetzt.

Die Verbindungen der Formeln I1 und I2 lassen sich wie folgt herstellen:

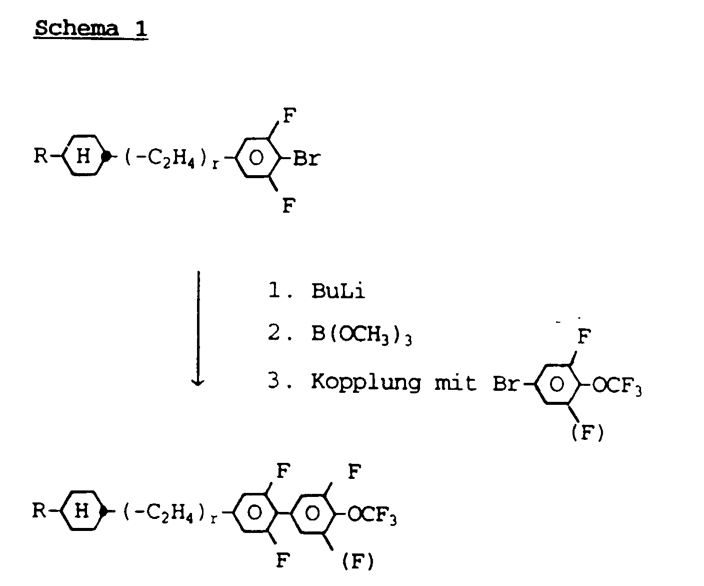

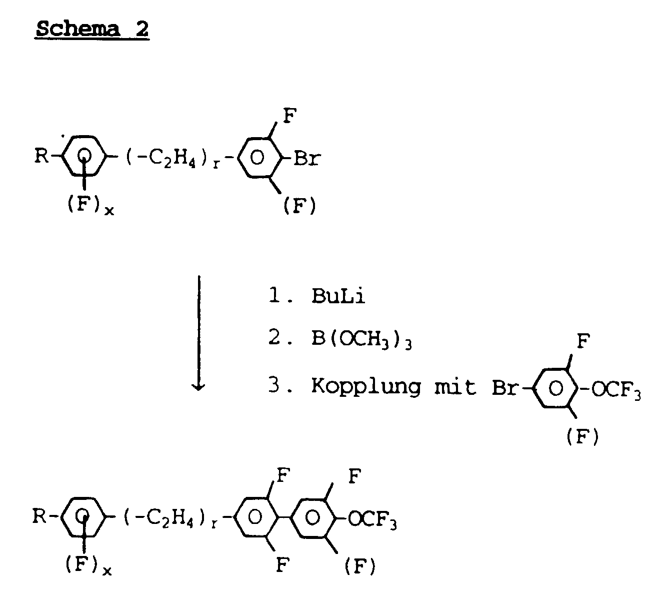

Verbindungen der Formel I, worin R OC₂F₅ bedeutet, lassen sich beispielsweise wie folgt herstellen:

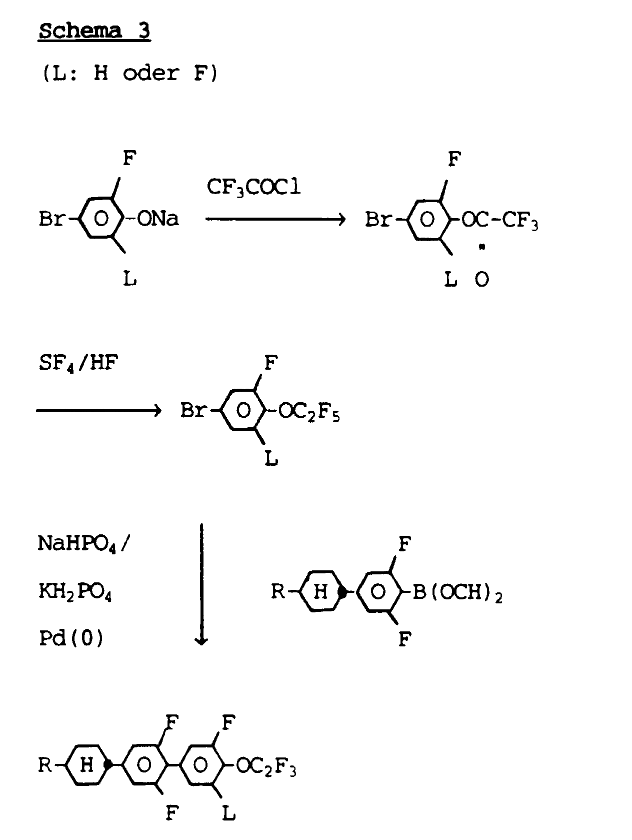

Weitere Synthesemethoden sind für den Fachmann augenscheinlich. Beispielsweise können in 5-Position entsprechend substituierte 1,3-Difluorbenzol-Verbindungen oder monofluorierte Analoga (L = H) gemäß obigem Schema in die 1,3-Difluor-Verbindungen oder monofluorierte Analoga (L = H) überführt werden und der Rest R-(A¹-Z¹)ₘ anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) eingeführt werden.

Die Verbindungen der Formel II∗ können beispielsweise nach folgenden Syntheseschemata hergestellt werden:

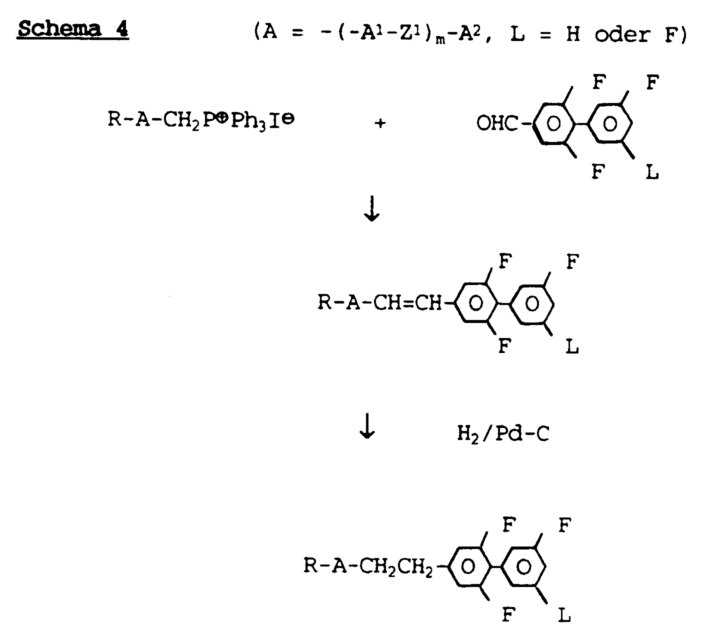

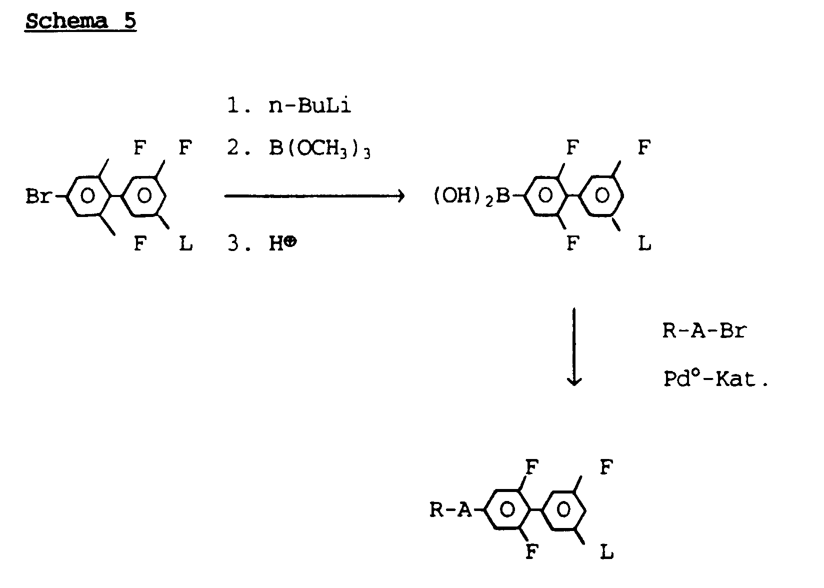

Die Ausgangsmaterialien sind entweder bekannt oder können in Analogie zu bekannten Verbindungen hergestellt werden.

Ester der-Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

In einem weiteren Verfahren zur Herstellung der Verbindungen der Formel I setzt man ein Arylhalogenid mit einem Olefin um in Gegenwart eines tertiären Amins und eines Palladiumkatalysators (vgl. R.F. Heck, Acc. Chem. Res. 12 (1979) 146).

Geeignete Arylhalogenide sind beispielsweise Chloride, Bromide und Iodide, insbesondere Bromide und Iodide. Die für das Gelingen der Kupplungsreaktion erforderlichen tertiären Amine, wie z.B. Triethylamin, eignen sich auch als Lösungsmittel. Als Palladiumkatalysatoren sind beispielsweise dessen Salze, insbesondere Pd(II)-acetat, mit organischen Phosphor(111)-Verbindungen wie z.B. Triarylphosphanen geeignet. Man kann dabei in Gegenwart oder Abwesenheit eines inerten Lösungsmittels bei Temperaturen zwischen etwa 0° und 150°, vorzugsweise zwischen 20° und 100°, arbeiten; als Lösungsmittel kommen z.B. Nitrile wie Acetonitrile oder Kohlenwasserstoffe wie Benzol oder Toluol in Betracht. Die als Ausgangsstoffe eingesetzten Arylhalogenide und Olefine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Auf diese Weise sind beispielsweise Stilbenderivate herstellbar. Die Stilbene können weiterhin hergestellt werden durch Umsetzung eines 4-substituierten Benzaldehyds mit einem entsprechenden Phoshorylid nach Wittig. Man kann aber auch Tolane der Formel I herstellen, indem man anstelle des Olefins monosubstituiertes Acetylen einsetzt (Synthesis 627 (1980) oder Tetrahedron Lett. 27, 1171 (1986)).

Weiterhin können zur Kopplung von Aromaten Arylhalogenide mit Arylzinnverbindungen umgesetzt werden. Bevorzugt werden diese Reaktionen unter Zusatz eines Katalysators wie z.B. eines Palladium(0)komplexes in inerten Lösungsmitteln wie Kohlenwasserstoffen bei hohen Temperaturen, z.B. in siedendem Xylol, unter Schutzgas durchgeführt.

Kopplungen von Alkinyl-Verbindungen mit Arylhalogeniden können analog dem von A.O. King, E. Negishi, F.J. Villani und A. Silveira in J. Org. Chem. 43, 358 (1978) beschriebenen Verfahren durchgeführt werden.

Tolane der Formel I können auch über die Fritsch-Buttenberg-Wiechell-Umlagerung (Ann. 279, 319, 1984) hergestellt werden, bei der 1,1-Diaryl-2-halogenethylene umgelagert werden zu Diarylacetylenen in Gegenwart starker Basen.

Tolane der Formel I können auch hergestellt werden, indem man die entsprechenden Stilbene bromiert und anschließend einer Dehydrohalogenierung unterwirft. Dabei kann man an sich bekannte, hier nicht naher erwähnte Varianten dieser Umsetzung anwenden.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zwecknäßig in einem inerten Lösungsmittel wie z. B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100 °C.

Die Verbindungen der Formel I, worin R = Alkenyl bedeutet, sind aus den entsprechenden Cyanverbindungen (R = CN) zugänglich, die mit Diisobutylaluminium in die Aldehyde (R = CHO) überführt werden. Beispielsweise kann sukzessiv eine Methylengruppe eingeführt werden durch Wittig-Reaktion des Aldehyds mit Methoxymethyl-triphenylphosphoniumchlorid und anschließende Hydrolyse des erhaltenen Enolethers (z.B. mit verdünnter Salzsäure).

Die Ausgangsmaterialien sind entweder bekannt oder können in Analogie zu bekannten Verbindungen hergestellt werden.

Die Verbindungen mit einer -(CH₂)₄-Brücke können nach folgendem Schema hergestellt werden:

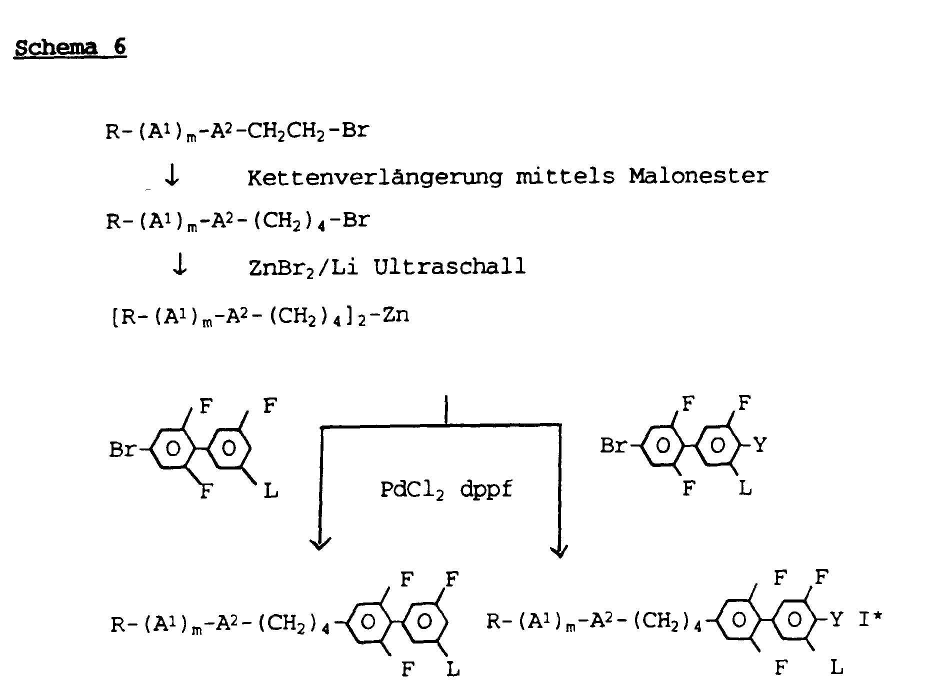

Bei der Pd(II)-katalysierten Kopplungsreaktion wird entweder direkt das Zielprodukt I^{∗} gebildet oder ein Vorprodukt, in das völlig analog zu den vorstehenden Methoden für Verbindungen oder Formel I der Rest -Y eingeführt wird.

Die Verbindungen mit einer -CH=CH-CH₂CH₂-Brücke können noch Wittig gemäß folgendem Schema hergestellt werden:

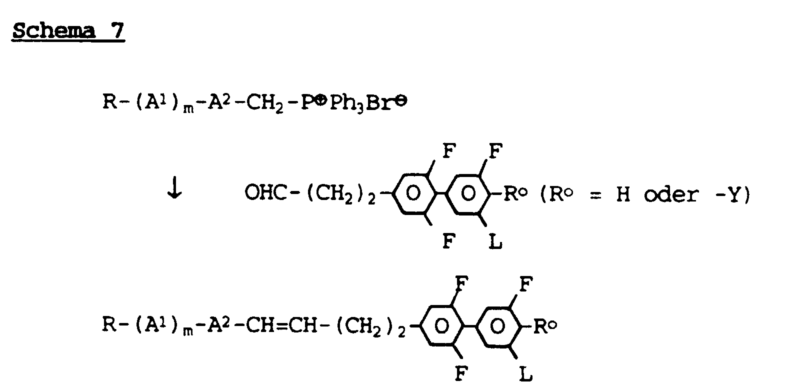

Gegenstand der Erfindung sind auch elektrooptische Anzeigen (insbesondere STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes.

Die erzielbaren Kombinationen aus Klarpunkt, Viskosität bei tiefer Temperatur, thermischer und UV-Stabilität und dielektrischer Anisotropie Übertreffen bei weitem bisherige Materialien aus dem Stand der Technik.

Die Forderung nach hohem Klarpunkt, nematischer Phase bei tiefer Temperatur sowie einem hohen Δε konnte bislang nur unzureichend erfüllt werden. Systeme wie z.B. ZLI-3119 weisen zwar vergleichbaren Klarpunkt und vergleichbar günstige Viskositäten auf, besitzen jedoch ein Δε von nur +3.

Andere Mischungs-Systeme besitzen vergleichbare Viskositäten und Werte von Δε, weisen jedoch nur Klarpunkte in der Gegend von 60 °C auf.

Die erfindungsgemäßen Flüssigkristallmischungen ermöglichen es bei Beibehaltung der nematischen Phase bis -20 °C und bevorzugt bis -30 °C, besonders bevorzugt bis -40 °C, Klarpunkte oberhalb 80°, vorzugsweise oberhalb 90°, besonders bevorzugt oberhalb 100 °C, gleichzeitig dielektrische Anisotropiewerte Δε ≥ 6, vorzugsweise ≥ 8 und einen hohen Wert für den spezifischen Widerstand zu erreichen, wodurch hervorragende STN- und MKF-Anzeigen erzielt werden können. Insbesondere sind die Mischungen durch kleine Operationsspannungen gekennzeichnet. Die TN-Schwellen (VIP) liegen unterhalb 2,0 V, vorzugsweise unterhalb 1,5 V, besonders bevorzugt < 1,3 V.

Es versteht sich, daß durch geeignete Wahl der Komponenten der erfindungsgemäßen Mischungen auch höhere Klarpunkte (z.B. oberhalb 110°) bei höheren Schwellenspannung oder niedrigere Klarpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringeren Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum eine kleinerere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Die Viskosität bei 20 °C ist vorzugsweise < 60 mPa.s, besonders bevorzugt < 50 mPa.s. Der nematische Phasenbereich ist vorzugsweise mindestens 90°, insbesondere mindestens 100°. Vorzugsweise erstreckt sich dieser Bereich mindestens von -20° bis +80°.

Messungen des "Capacity Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, daß erfindungsgemäße Mischungen enthaltend Verbindungen der Formel I eine deutlich kleinere Abnahme des HR mit steigender Temperatur aufweisen als analoge Mischungen enthaltend anstelle den Verbindungen der Formel I Cyanophenylcyclohexane der Formel

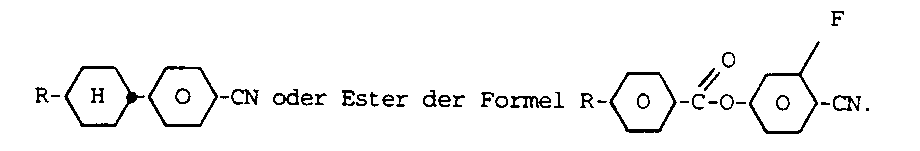

Auch die UV-Stabilität der erfindungsgemäßen Mischungen ist erheblich besser, d. h. sie zeigen eine deutlich kleinere Abnahme des HR unter UV-Belastung.

Vorzugsweise basieren die erfindungsgemäßen Medien auf mehreren (vorzugsweise zwei oder mehr) Verbindungen der Formel I, d.h. der Anteil dieser Verbindungen ist 5-95 %, vorzugsweise 10-60 % und besonders bevorzugt im Bereich von 20-50 %.

Die einzelnen Verbindungen der Formeln I bis XVI und deren Unterformeln, die in den erfindungsgemäßen Medien verwendet werden können, sind entweder bekannt, oder sie können analog zu den bekannten Verbindungen hergestellt werden.

Bevorzugte Ausführungsformen sind im folgenden angegeben:
- halogeniert bedeutet bei X in Formel I fluoriert und/ oder chloriert, vorzugsweise jedoch fluoriert
- X ist vorzugsweise OCF₃, OCF₂H, OC₂F₅ oder OCH = CF₂, oder -O-Q-Y, worin Q unsubstituiertes oder ein- oder mehrfach durch Fluor und/oder Chlor substituiertes Alkylen oder Alkenylen mit 1 bis 5 C-Atomen und Y Hal, CHal₃ oder CHHal₂ bedeutet und Hal F oder Cl, vorzugsweise F ist,
- Q ist vorzugsweise -CH₂-, -CH₂CH₂-, -CHF-, -CF₂-, -CH₂CHF-, -CHFCH₂-, -CH₂CHF-, -CF₂CH₂-, -CH₂-CH₂-, -CF₂CF₂-, -CH₂CF₂-, -CH=CH-, -CH=CF-, -CF=CF-, -CH=CCl-, -CH₂CH=CF-, -CH₂-CF=CF- oder -CF₂-CF=CF-,
- Die Verbindung der Formel IV ist vorzugsweise 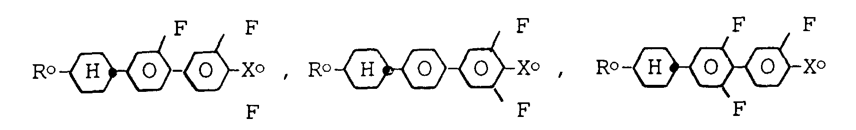 oder 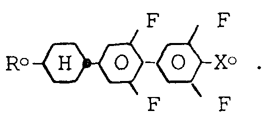
- Medium enthält zusätzlich eine oder mehrere Verbindungen ausgewählt aus der Gruppe bestehend aus den allgemeinen Formeln VI bis XII: 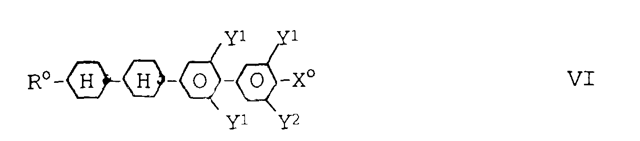 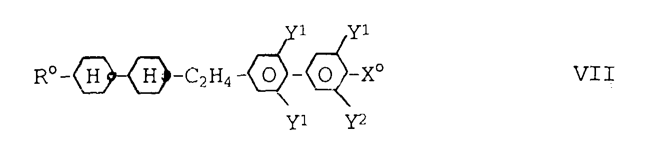 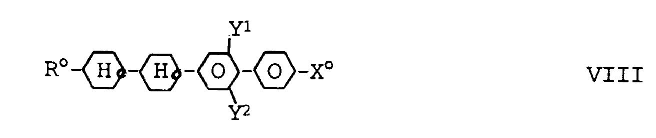 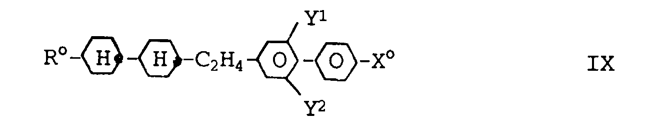 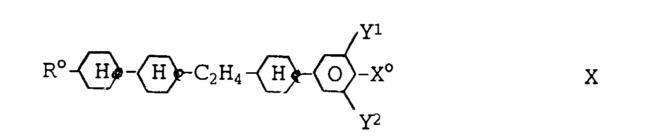 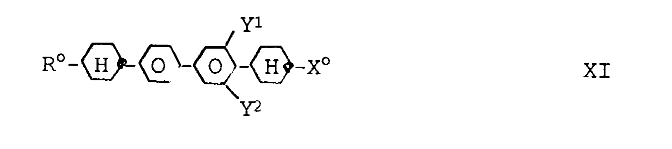 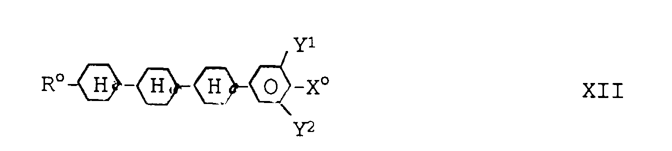 worin R^{o}, 4 Y¹ und Y² jeweils unabhängig voneinander eine der in Anspruch 1 angegebene Bedeutung haben und X^{o} F, Cl, CF₃, OCF₃, OCHF₂, Alkyl, Oxaalkyl, Fluoralkyl oder Alkenyl mit jeweils bis zu 7C-Atomen bedeutet.
- Der Anteil an Verbindungen der Formeln I bis V zusammen beträgt im Gesamtgemisch mindestens 50 Gew.-%
- der Anteil an Verbindungen der Formel I beträgt im Gesamtgemisch 3 bis 80 Gew.-%
- der Anteil an Verbindungen der Formeln II bis V im Gesamtgemisch beträgt 20 bis 80 Gew.-%
- 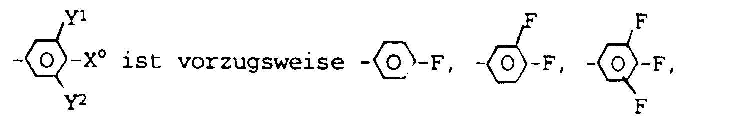 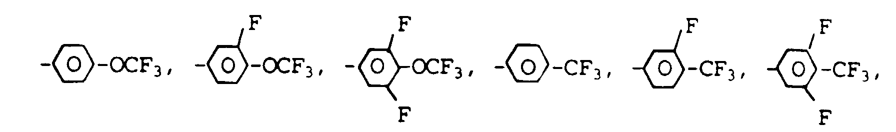 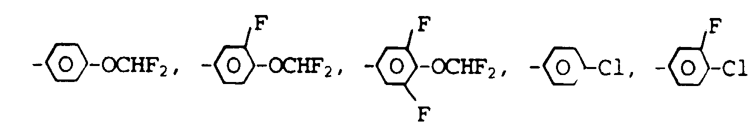 oder 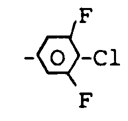
- das Medium enthält Verbindungen der Formeln II, III, IV oder V
- R^{o} ist geradkettiges Alkyl oder Alkenyl mit 2 bis 7 C-Atomen
- das Medium besteht im wesentlichen aus Verbindungen der Formeln I bis V
- das Medium enthält weitere Verbindungen, vorzugsweise ausgewählt aus der folgenden Gruppe bestehend aus den allgemeinen Formeln XIII bis XVII: 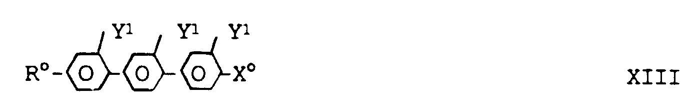 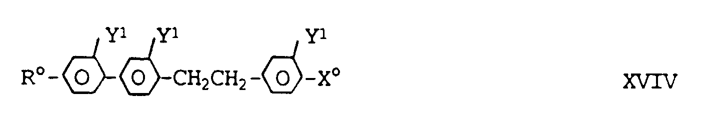 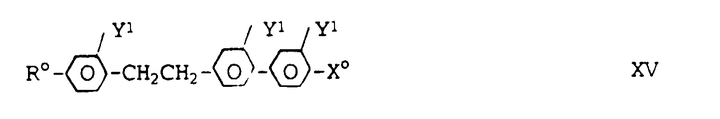 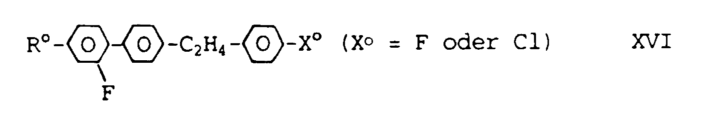 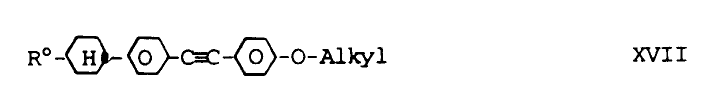 worin R^{o} und X^{o} die in Anspruch 2 angegebene Bedeutung haben und die 1,4-Phenylenringe durch CN, Chlor oder Fluor substituiert sein können. Vorzugsweise sind die 1,4-Phenylenringe ein- oder mehrfach durch Fluoratome substituiert.
- Das Gewichtsverhältnis I: (II + III + IV + V) ist vorzugsweise 1 : 10 bis 10 : 1.
- Medium besteht im wesentlichen aus Verbindungen ausgewählt aus der Gruppe bestehend aus den allgemeinen Formeln I bis XII.

Es wurde gefunden, daß bereits ein relativ geringer Anteil an Verbindungen der Formel I im Gemisch mit üblichen Flüssigkristallmaterialien, insbesondere jedoch mit einer oder mehreren Verbindungen der Formel II, III, IV und/oder V zu einer beträchtlichen Erniedrigung der Schwellenspannung und zu niedrigen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Die Verbindungen der Formeln I bis V sind farblos, stabil und untereinander und mit anderen Flüssigkristallmaterialien gut mischbar.

Der Ausdruck "Alkyl" umfaßt geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" umfaßt geradkettige und verzweigte Alkenylgruppen mit 2-7 Kohlenstoffatomen, insbesondere die geradkettigen Gruppen. Besonders Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigen Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" umfaßt vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n = 1 und m 1 bis 6.

Durch geeignete Wahl der Bedeutungen von R^{o} und X^{o} können die Ansprechzeiten, die Schwellenspannung, die Steilheit der Transmissionskennlinien etc. in gewünschter Weise modifiziert werden. Beispielsweise führen 1E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten.

Eine Gruppe -CH₂CH₂- in Z¹ führt im allgemeinen zu höheren Werten von k₃₃/k₁₁ im Vergleich zu einer einfachen Kovalenzbindung. Höhere Werte von k₃₃/k₁₁ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt.

Das optimale Mengenverhältnis der Verbindungen der Formeln I und II + III + IV + V hängt weitgehend von den gewünschten Eigenschaften, von der Wahl der Komponenten der Formeln I, II, III, IV und/oder V und von der Wahl weiterer gegebenenfalls vorhandener Komponenten ab. Geeignete Mengenverhältnisse innerhalb des oben angegebenen Bereichs können von Fall zu Fall leicht ermittelt werden.

Die Gesamtmenge an Verbindungen der Formeln I bis XVI in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I bis XI ist.

In einer besonders bevorzugten Ausführungsform enthalten die erfindungsgemäßen Medien Verbindungen der Formel II bis V (vorzugsweise II und/oder III), worin X^{o} OCF₃, CCHF₂, F, OCH=CF₂, OCF=CF₂ oder OCF₂-CF₂H bedeutet. Eine günstige synergistische Wirkung mit den Verbindungen der Formel I führt zu besonders vorteilhaften Eigenschaften.

Für STN-Anwendungen enthalten die Medien vorzugsweise Verbindungen ausgewählt aus der Gruppe bestehend aus den Formeln II bis V, worin X^{o} vorzugsweise OCHF₂ oder CN bedeutet.

Die erfindungsgemäßen Medien können ferner eine Komponente A enthalten bestehend aus einer oder mehreren Verbindungen mit einer dielektrischen Anisotropie von -1,5 bis +1,5 der allgemeinen Formel I' 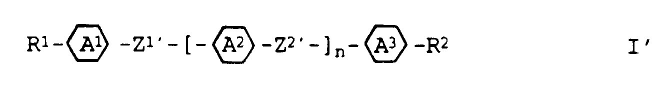 worin
- R¹ und R²: jeweils unabhängig voneinander n-Alkyl, n-Alkoxy, ω-Fluoralkyl oder n-Alkenyl mit bis zu 9 C-Atomen, 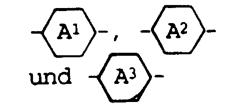
jeweils unabhängig voneinander 1,4-Phenylen, 2- oder 3-Fluor-1,4-phenylen, trans-1,4-Cyclohexylen oder 1,4-Cyclohexenylen,
- Z¹'und Z²': jeweils unabhängig voneinander -CH₂CH₂-, -C≡C-, -CO-O-, -O-CO-, oder eine Einfachbindung,
und
- n: 0, 1 oder 2
bedeutet.

Komponente A enthält vorzugsweise eine oder mehrere Verbindungen ausgewählt aus der Gruppe bestehend aus II1 bis II7: 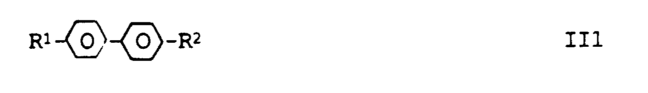 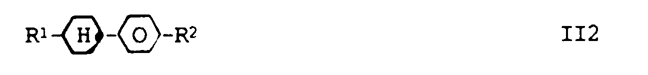 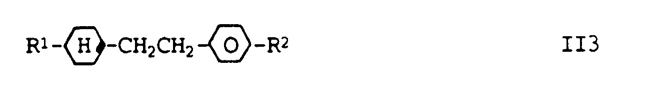 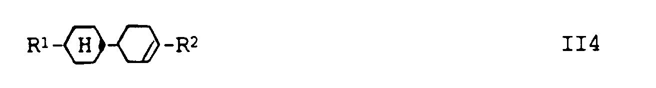 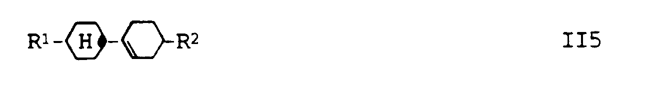 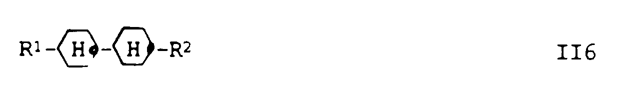 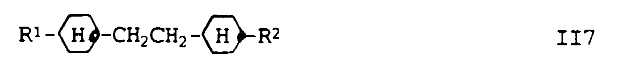 worin R¹ und R² die bei Formel I' angegebene Bedeutung haben.

Vorzugsweise enthält Komponente A zusätzlich eine oder mehrere Verbindungen ausgewählt aus der Gruppe bestehend aus II8 bis II20: 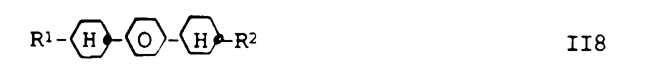 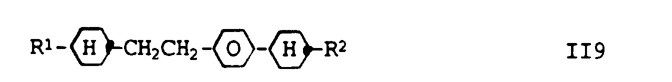 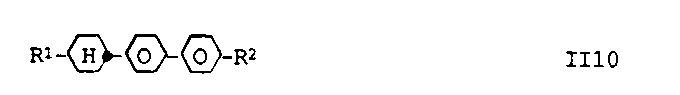 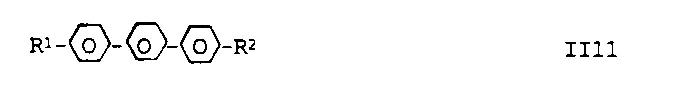 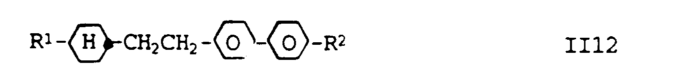 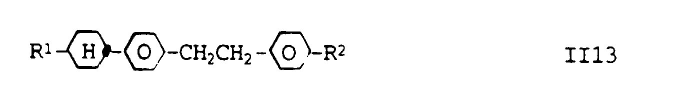 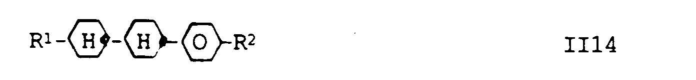 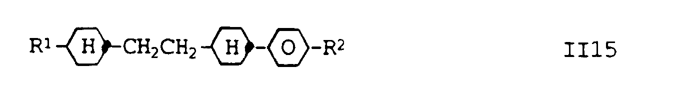 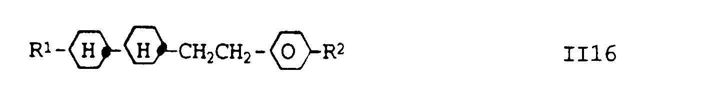 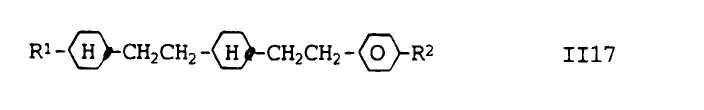 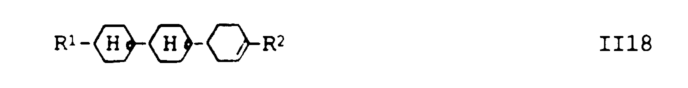 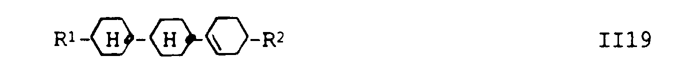 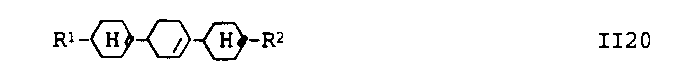 worin R¹ und R² die bei Formel I' angegebene Bedeutung haben und die 1,4-Phenylengruppen in II8 bis II17 jeweils unabhängig voneinander auch durch Fluor ein- oder mehrfach substituiert sein können.

Ferner enthält Komponente A vorzugsweise zusätzlich eine oder mehrere Verbindungen ausgewählt aus der Gruppe bestehend aus 1121 bis II25 enthält: 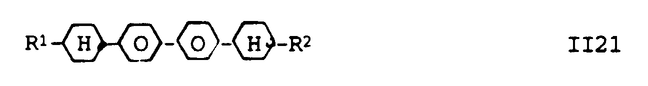 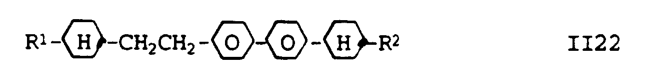 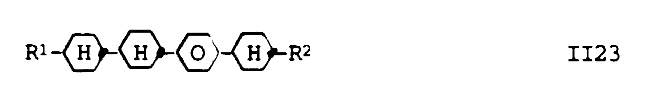 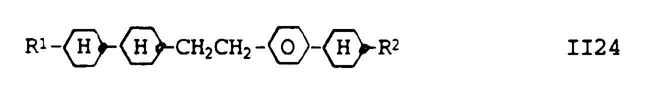 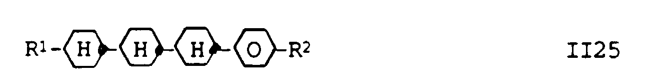 worin R¹ und R² die bei Formel I' angegebene Bedeutung haben und die 1,4-Phenylengruppen in II21 bis II25 jeweils unabhängig voneinander auch durch Fluor ein- oder mehrfach substituiert sein können.

Schließlich sind derartige Mischungen bevorzugt, deren Komponente A eine oder mehrere Verbindungen ausgewählt aus der Gruppe bestehend aus II26 und II27 enthält: 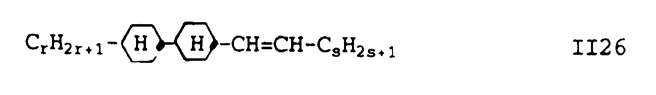 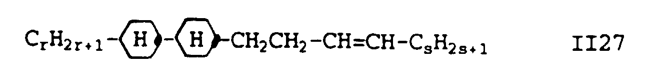 worin CᵣH₂ᵣ₊₁ eine geradkettige Alkylgruppe mit bis zu 7 C-Atomen und s 0, 1, 2 oder 3 ist.

In einigen Fällen erweist sich der Zusatz von Verbindungen der Formel 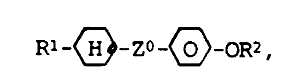 worin

R¹ und R² die bei Formel I' angegebene Bedeutung haben, und Z⁰ eine Einfachbindung, -C₂H₄-, 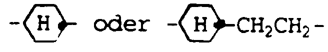 bedeuten, zur Unterdrückung smektischer Phasen als vorteilhaft, obwohl hierdurch der spezifische Widerstand erniedrigt werden kann. Zur Erzielung von für die Anwendung optimaler Parameterkombinationen kann der Fachmann leicht feststellen, ob und falls ja, in welcher Menge diese Verbindungen zugesetzt sein können. Normalerweise werden weniger als 15 %, insbesondere 5-10 % verwendet.

Ferner bevorzugt sind Flüssigkristallmischungen, die eine oder mehrere Verbindungen ausgewählt aus der Gruppe bestehend aus III' und IV' enthalten: 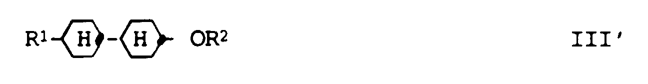 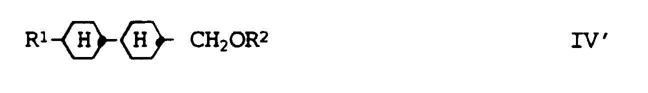 worin
R¹ und R² die bei Formel I' angegebene Bedeutung haben.

Die Art und Menge der polaren Verbindungen mit positiver dielektrischer Anisotropie ist an sich nicht kritisch. Der Fachmann kann unter einer großen Palette bekannter und in vielen Fällen auch kommerziell verfügbarer Komponenten und Basisgemische in einfachen Routineversuchen geeignete Materialien auswählen. Vorzugsweise enthalten die erfindungsgemäßen Medien eine oder mehrere Verbindungen der Formel I"' 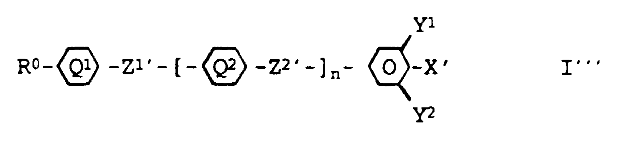 worin Z¹', Z²' und n die bei Formel I"' angegebene Bedeutung haben, 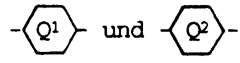 jeweils unabhängig voneinander 1,4-Phenylen, trans-1,4-Cyclohexylen oder 3-Fluor-1,4-phenylen oder einer der Reste 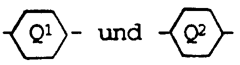 auch trans-1,3-Dioxan-2,5-diyl, Pyrimidin-2,5-diyl, Pyridin-2,5-diyl oder 1,4-Cyclohexenylen bedeutet, R^{o} n-Alkyl, n-Alkenyl, n-Alkoxy oder n-Oxaalkyl mit jeweils bis zu 9 C-Atomen, Y¹ oder Y² H oder F und X' CN, Halogen, CF₃, OCF₃ oder OCHF₂ ist.

In einer bevorzugten Ausführungsform basieren die erfindungsgemäßen Medien für STN- oder TN-Anwendungen auf Verbindungen der Formel I"' worin X' CN bedeutet. Es versteht sich, daß auch kleinere oder größere Anteile von anderen Verbindungen der Formel I" (X' ≠ CN) in Frage kommen. Für MFK-Anwendungen enthalten die erfindungsgemäßen Medien vorzugsweise nur bis zu ca. 10 % an Nitrilen der Formel I" (vorzugsweise jedoch keine Nitrile der Formel I", sondern Verbindungen der Formel I' mit X' = Halogen, CF₃, OCF₃ oder OCHF₂). Diese Medien basieren vorzugsweise auf den Verbindungen der Formeln II bis XVI .

Vorzugsweise enthalten die erfindungsgemäßen Medien ein oder mehrere Verbindungen mit einer dielektrischen Anisotropie im Bereich von -6 ≤ Δε ≤ -1,5 der Formel 1'" 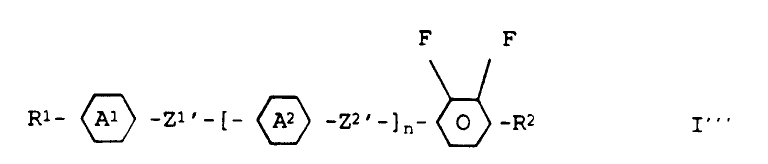 worin R¹, 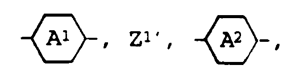 Z²', n und R² die bei Formel I' angegebene Bedeutung haben, für Anwendungen, in denen eine kleine Änderung der Kapazität des Pixels beim Schalten erwünscht ist (z.B. MIM-Displays oder TFT-Displays).

Vorzugsweise werden Verbindungen der Formeln I^{1"'} bis I^{3"'} verwendet:

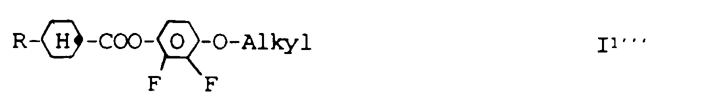

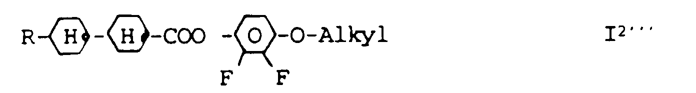

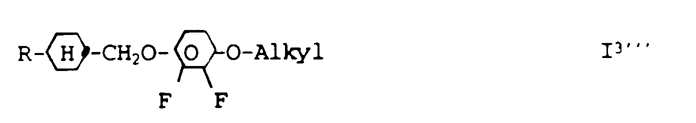

Der Aufbau der erfindungsgemäßen STN-bzw. MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefaßt und umfaßt auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze enthalten. Beispielsweise können 0-15 % pleochroitische Farbstoffe oder chirale Dotierstoffe zugesetzt werden.

C bedeutet eine kristalline, S eine smektische, S_{B} eine smektisch B, N eine nematische und I die isotrope Phase.

V₁₀ bezeichnet die Spannung für 10 % Transmission (Blickrichtung senkrecht zur Plattenoberfläche). tₒₙ bezeichnet die Einschaltzeit und t_{off} die Ausschaltzeit bei einer Betriebsspannung entsprechend dem 2,5-fachen Wert von V₁₀. Δn bezeichnet die optische Anisotropie und nₒ den Brechungsindex. Δε bezeichnet die dielektrische Anisotropie (Δε = ε_{||}- ε_{⊥} wobei ε_{||} die Dielektrizitatskonstante parallel zu den Moleküllängsachsen und ε_{⊥} die Dielektrizitätskonstante senkrecht dazu bedeutet). Die elektrooptischen Daten wurden in einer TN-Zelle im 1. Minimum (d.h. bei einem d Δn-Wert von 0,5) bei 20 °C gemessen, sofern nicht ausdrücklich etwas anderes angegeben wird. Die optischen Daten wurden bei 20 °C gemessen, sofern nicht ausdrücklich etwas anderes angegeben wird.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁ -O-CₛH₂ₛ - | CN | H | H |
| nAm | CₙH₂ₙ₊₁ | COOCₘH₂ₘ₊₁ | H | H |
| nOCCF₂.F.F | CₙH₂ₙ₊₁ | OCH₂CF₂H | F | F |
| nOCF₂.F.F | CₙH₂ₙ₊₁ | OCHF₂ | F | F |
| nCl.F.F | CₙH₂ₙ₊₁ | Cl | F | F |
| nOCF3.F.F | CₙH₂ₙ₊₁ | OCF₃ | F | F |

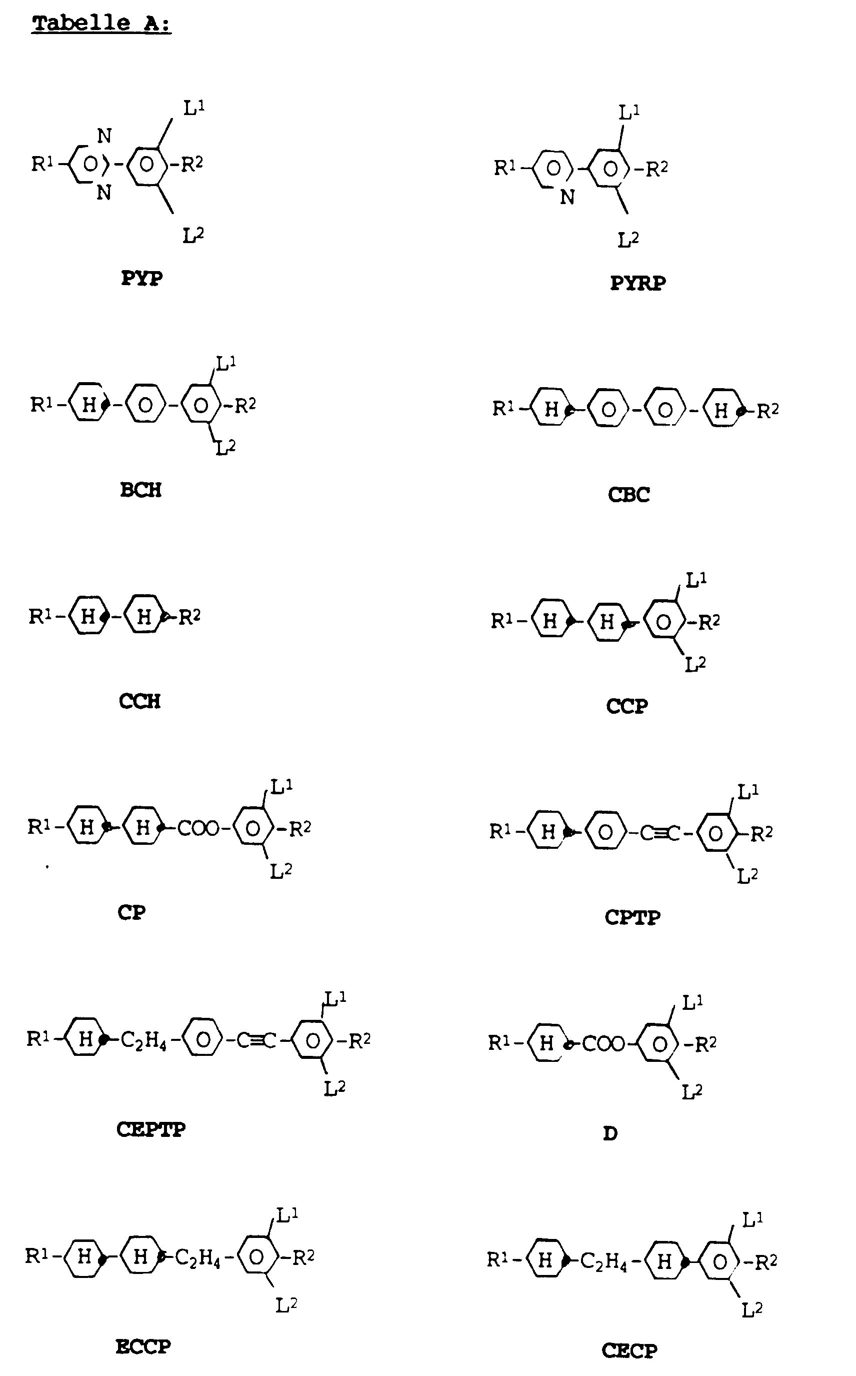

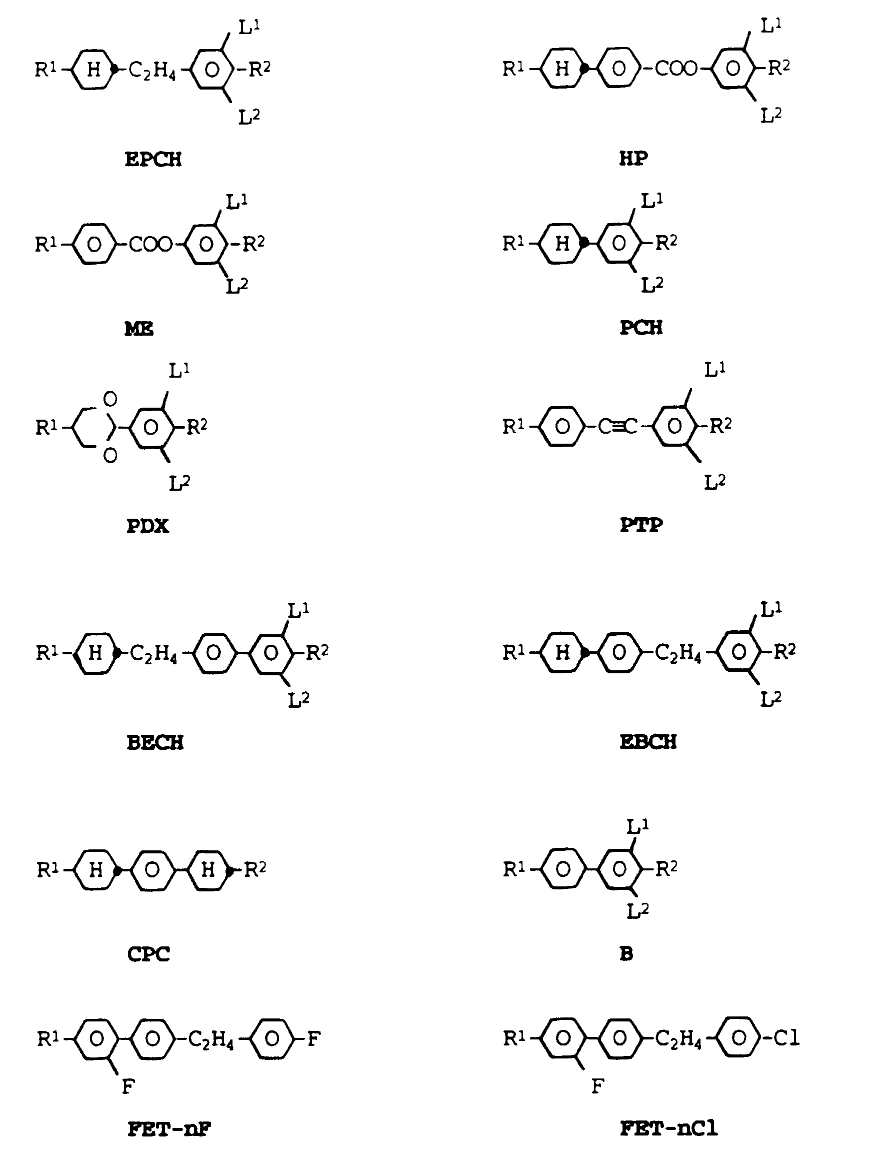

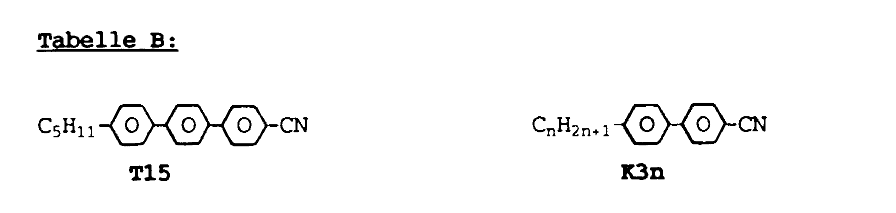

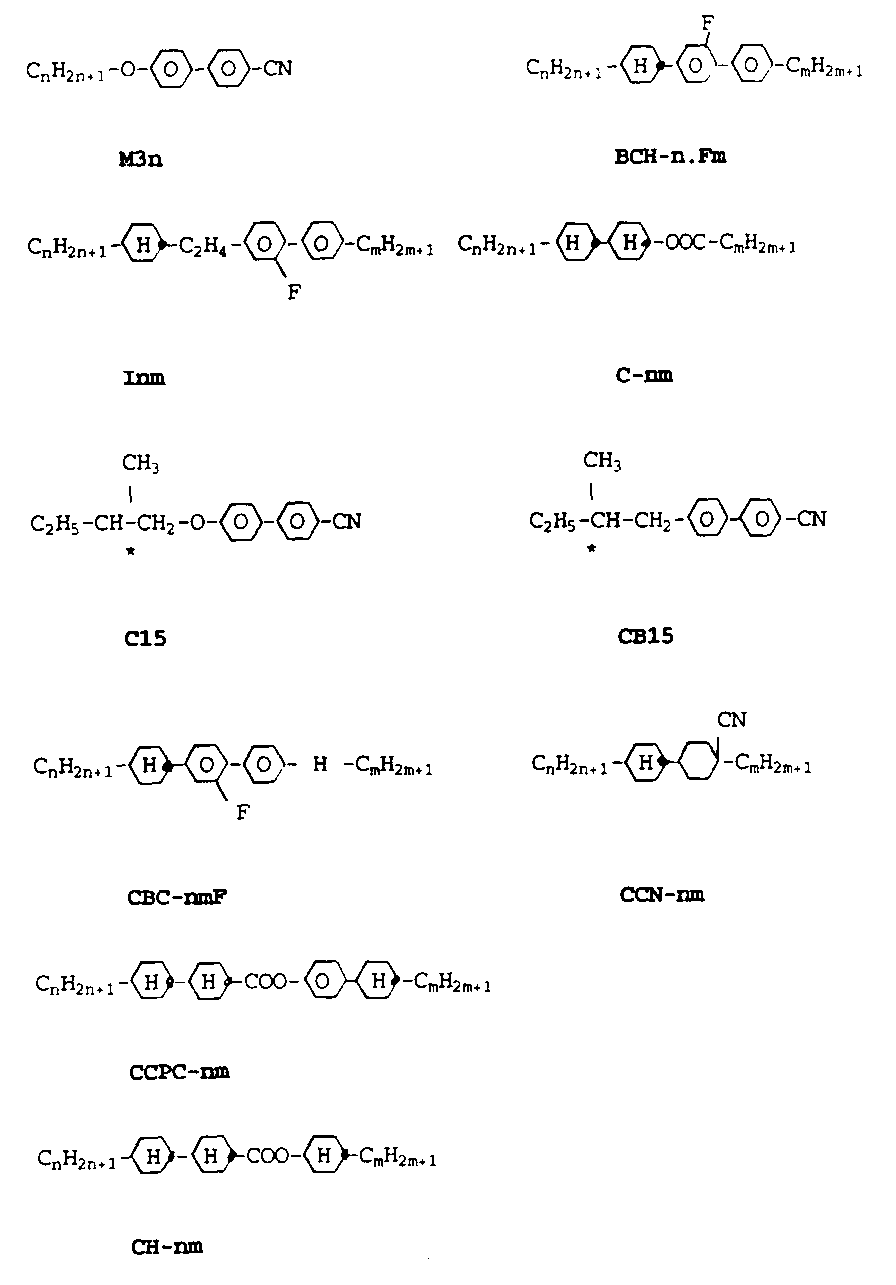

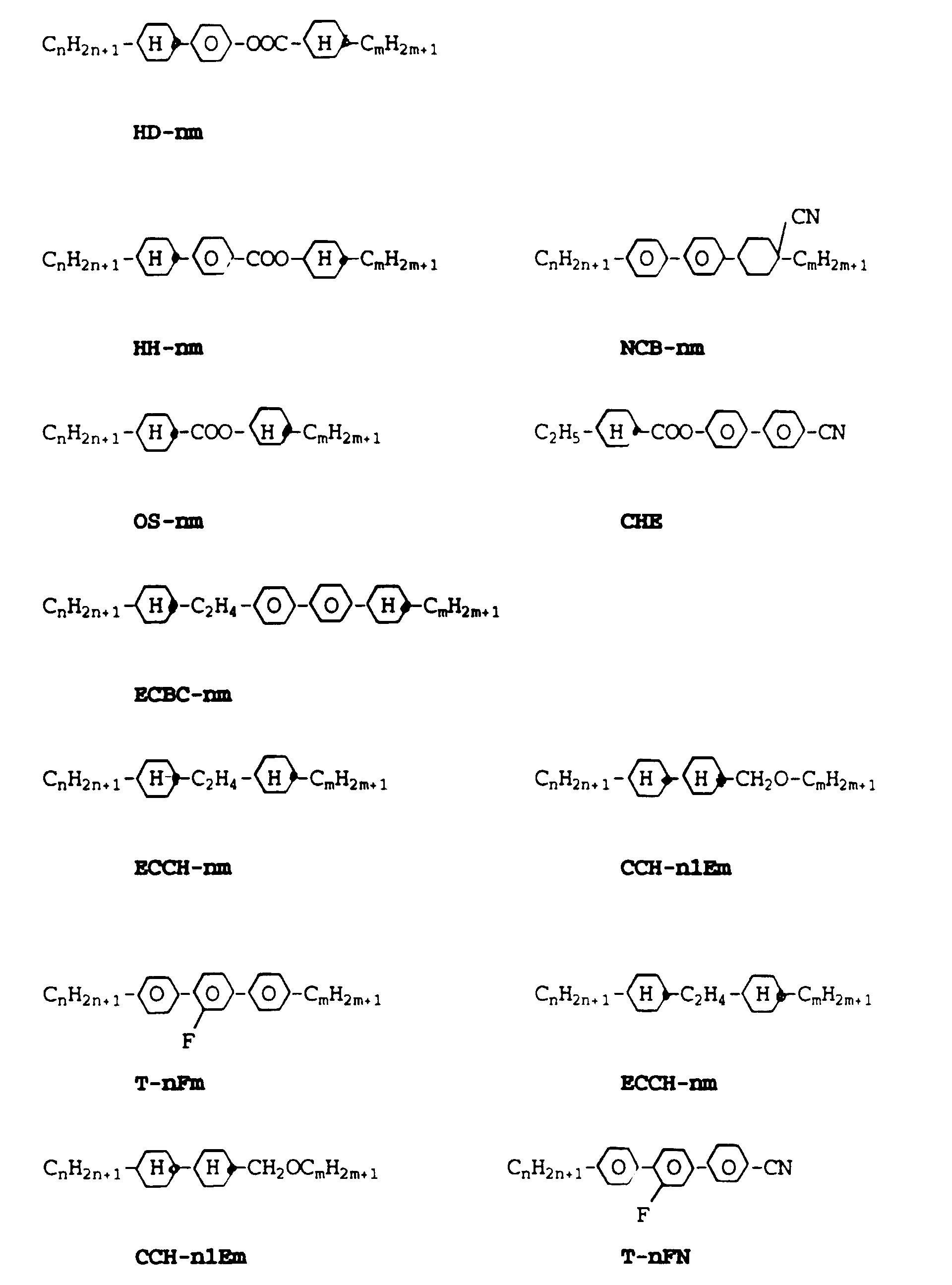

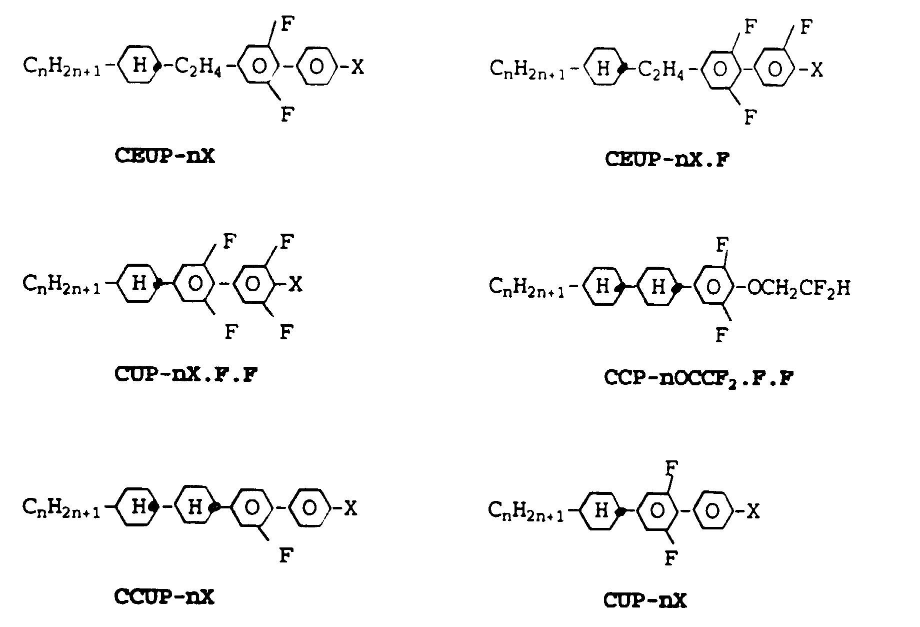

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klarpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskositat (mm²/sec) wurde bei 20 °C bestimmt.

"Übliche Aufarbeitung" bedeutet: man gibt gegebenenfalls Wasser hinzu, extrahiert mit Dichlormethan, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DMEU: 1,3-Dimethyl-2-imidazolidinon
- KOT: Kalium-tertiär-butanolat
- THF: Tetrahydrofuran
- pTSOH: p-Toluolsulfonsäure

### Beispiel 1

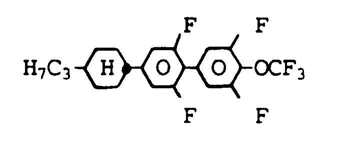

0,05 mol 4-Brom-2,6-difluor-trifluormethoxybenzol, 0,05 mol 4-(trans-4-n-Propylcyclohexyl)-2,6-difluorphenylboronsäure und 1 g Tetrakistriphenylphosphinpalladium(0)-Katalysator werden in 100 ml Toluol und 40 ml Ethanol gelost und mit 50 ml 2 M Na₂CO₃-Lösung versetzt. Man kocht 4 h am Rückfluß und arbeitet extraktiv auf. Nach chromatographischer Aufreinigung und Kristallisation erhält man das Zielprodukt: K 68 I; Δε = 19,26; Δn = 0,105.

Analog werden die folgenden Verbindungen der Formel 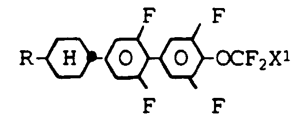 hergestellt:

| R | L | X¹ | |
|---|---|---|---|
| H₃C | H | Cl | |
| H₃C | F | Cl | |
| H₅C₂ | H | Cl | |
| H₅C₂ | F | Cl | |
| H₇C₃ | H | Cl | |
| H₇C₃ | F | Cl | |
| H₉C₄ | H | Cl | |
| H₉C₄ | F | Cl | |
| H₁₁C₅ | H | Cl | |
| H₁₁C₅ | F | Cl | |
| H₁₃C₆ | H | Cl | |
| H₁₃C₆ | F | Cl | |
| H₁₅C₇ | H | Cl | |
| H₁₅C₇ | F | Cl | |
| H₃C | H | H | |
| H₃C | F | H | |
| H₅C₂ | H | H | |
| H₅C₂ | F | H | |
| H₇C₃ | H | H | |
| H₇C₃ | F | H | |
| H₉C₄ | H | H | |
| H₉C₄ | F | H | |
| H₁₁C₅ | H | H | |
| H₁₁C₅ | F | H | |
| H₁₃C₆ | H | H | |
| H₁₃C₆ | F | H | |
| H₁₅C₇ | H | H | |
| H₁₅C₇ | F | H | |
| H₃C | H | F | |
| H₃C | F | F | K 69 I; Δn = + 0,0857; Δε = 19,33 |
| H₅C₂ | H | F | |
| H₅C₂ | F | F | K 78 I; Δn = + 0,0878; Δε = 18,86 |
| H₇C₃ | H | F | |
| H₉C₄ | H | F | |
| H₉C₄ | F | F | |
| H₁₁C₅ | H | F | |
| H₁₁C₅ | F | F | K 78 I; Δn = + 0,1014; Δε = 18,74 |
| H₁₃C₆ | H | F | |
| H₁₃C₆ | F | F | |
| H₁₅C₇ | H | F | |
| H₁₅C₇ | F | F | |

### Beispiel 2

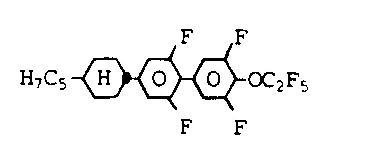

### Schritt 2.1

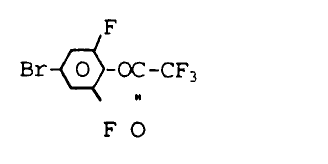

Unter Schutzgas werden 0,43 mol 4-Brom-2,6-difluornatriumphenolat in 1000 ml THF gelöst und auf -60 °C abgekühlt. Innerhalb von 0,5 h werden 0,44 mol Trifluoressigsäurechlorid in die Lösung eingeleitet. Anschließend wird bei -60 °C 1 h gerührt. Man läßt auf 10 °C erwärmen und engt die Lösung ein. Der Rückstand wird in 1000 ml Hexan aufgenommen und 0,5 h gerührt. Abschließend wird das Lösungsmittel am Rotationsverdampfer entfernt. Nach Zugabe von 500 ml Dichlormethan wird wie üblich aufgearbeitet.

### Schritt 2.2

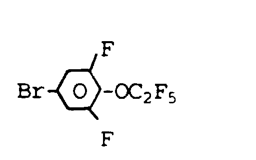

0,05 mol Trifluoracetat (Schritt 2.1) werden vorgelegt und analog I.L. Knunyants, G.G. Yakolson, Syntheses of Fluoroorganic Compounds S. 267 mit SF₄/HF versetzt. Das Gemisch wird im Autoklaven 3 h bei 150 °C und 6 h bei 175 °C erhitzt. Dann läßt man abkühlen und leitet die gasförmigen Produkte in eine Alkalilösung. Der Rückstand wird nach Wasserdampfdestillation mit Ether versetzt und anschließend wir üblich aufgearbeitet.

### Schritt 2.3

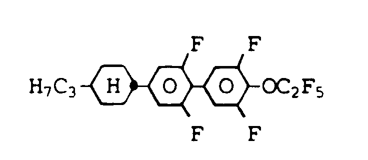

0,02 mol Pentafluorethoxy-2,6-difluorbrombenzol werden in einer N₂-Atmosphäre in 75 ml THF gelöst und unter Rühren auf 60 °C erwärmt. Eine Lösung bestehend aus 0,037 mol Kaliumdihydrogenphosphat und 0,074 mol Natriumhydrogenphosphat in 40 ml Wasser und 0,46 g Tetrakis(triphenyl)phosphin und 4-(trans-4-n-Propylcyclohexyl)-2,6-difluorphenylboronsäure werden zugegeben und das Gemisch wird 24 h unter Rückfluß gekocht. Man läßt das Reaktionsgemisch auf Raumtemperatur abkühlen. Nach Zugabe von Methyl-tert.-Butylether wird wie üblich aufgearbeitet. K 78 N (72,2) I; Δn = 0,116; Δε = 18,88.

Analog werden die folgenden Verbindungen hergestellt:

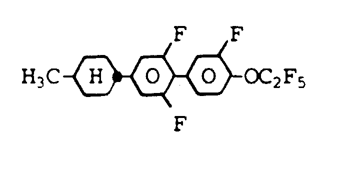

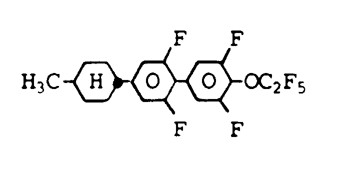

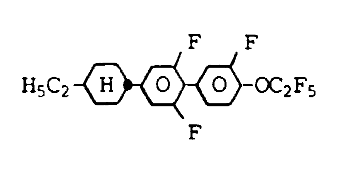

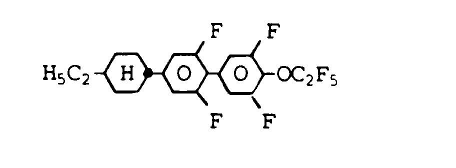

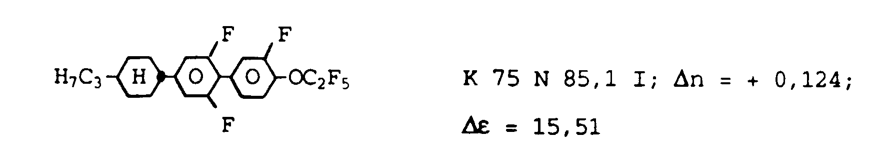

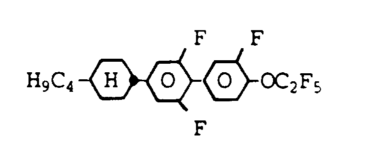

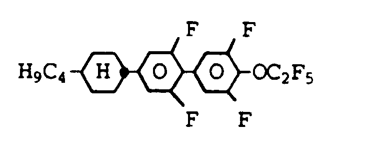

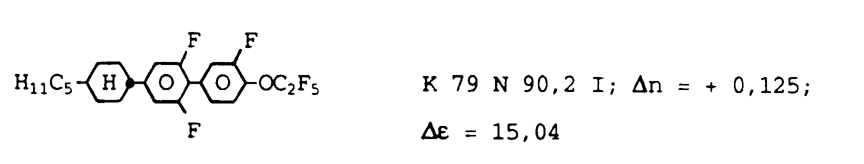

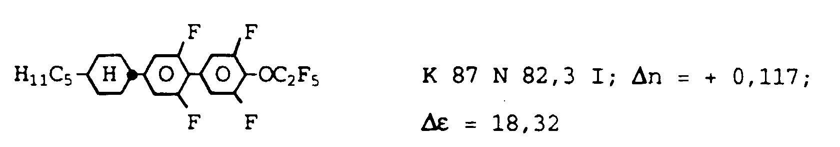

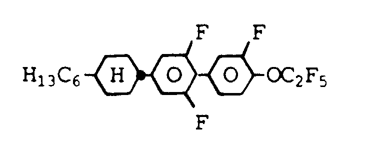

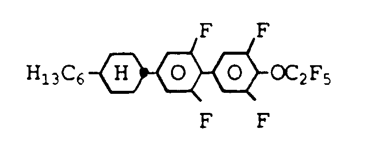

### Mischungsbeispiele

| Beispiel 1 | | | |
|---|---|---|---|
| PCH-6F | 3,5 % | Klärpunkt [°C] | 84 |
| PCH-7F | 3,0 % | Viskosität bei | |
| CCP-2OCF₂.F.F | 16,0 % | 20 °C [mm²s⁻¹] | 46 |
| CCP-3OCF₂.F.F | 15,0 % | Δn [20 °C, 589 nm] | 0,1017 |
| CCP-5OCF₂.F.F | 16,0 % | V_{(10,0,20)} [V] | 1,16 |
| CUP-2F.F | 6,0 % | V_{(50,0,20)} [V] | 1,45 |
| CUP-3F.F | 6,0 % | V_{(90,0,20)} [V] | 1,82 |
| CUP-5F.F | 5,0 % | | |
| CUP-2OCF₂.F.F | 6,0 % | | |
| CUP-3OCF₂.F.F | 6,0 % | | |
| CUP-5OCF₂.F.F | 6,0 % | | |
| CBC-33F | 3,5% | | |
| CBC-53F | 4,0 % | | |
| CBC-55F | 4,0 % | | |

| Beispiel 2 | | | |
|---|---|---|---|
| PCH-5F | 3,0 % | S → N [°C] | |
| CCP-2OCF₂.F.F | 16,0 % | Klärpunkt [°C] | +103 |
| CCP-3OCF₂.F.F | 15,0 % | Δn [589 nm, 20 °C] | +0,1086 |
| CCP-5OCF₂.F.F | 16,0 % | V_{(10,0,20)} [V] | 1,26 |
| CUP-2F.F | 5,0 % | V_{(90,0,20)} [V] | 2,04 |
| CUP-3F.F | 5,0 % | | |
| CUP-5F.F | 5,0 % | | |
| CUP-3OCF₂.F.F | 10,0 % | | |
| CUP-5OCF₂.F.F | 10,0 % | | |
| CBC-33F | 5,0 % | | |
| CBC-53F | 5,0 % | | |
| CBC-55F | 5,0 % | | |

| Beispiel 3 | | | |
|---|---|---|---|
| PCH-5F | 3,0 % | S → N [°C] | |
| CCP-2OCF₂.F.F | 16,0 % | Klärpunkt [°C] | +100 |
| CCP-3OCF₂.F.F | 15,0 % | Δn [589 nm, 20 °C] | +0,1077 |
| CCP-5OCF₂.F.F | 16,0 % | Δε [1 kHz, 20 °C] | |
| CUP-2F.F | 6,0 % | V_{(10,0,20)} [V] | 1,23 |
| CUP-3F.F | 6,0 % | V_{(90,0,20)} [V] | 1,95 |
| CUP-5F.F | 5,0 % | | |
| CUP-2OCF₂.F.F | 6,0 % | | |
| CUP-3OCF₂.F.F | 6,0 % | | |
| CUP-5OCF₂.F.F | 6,0 % | | |
| CBC-33F | 5,0 % | | |
| CBC-53F | 5,0 % | | |
| CBC-55F | 5,0 % | | |

| Beispiel 4 | | | |
|---|---|---|---|
| PCH-6F | 4,0 % | Klärpunkt [°C ] | +78 |
| PCH-7F | 4,0 % | | |
| CCP-2OCF₂.F.F | 16,0 % | | |
| CCP-3OCF₂.F.F | 15,0 % | | |
| CCP-5OCF₂.F.F | 16,0 % | | |
| CUP-2F.F | 6,0 % | | |
| CUP-3F.F | 6,0 % | | |
| CUP-5F.F | 5,0 % | | |
| CUP-2OCF₂.F.F | 6,0 % | | |
| CUP-3OCF₂.F.F | 6,0 % | | |
| CUP-5OCF₂.F.F | 6,0 % | | |
| CBC-33F | 3,0 % | | |
| CBC-53F | 3,0 % | | |
| CBC-55F | 4,0 % | | |

| Beispiel 5 | | | |
|---|---|---|---|
| PCH-6F | 3,5 % | S → N [°C] | < -40 |
| PCH-7F | 3,0 % | Klärpunkt [°C] | +84 |
| CCP-2OCF₂.F.F | 16,0 % | Δn [589 nm, 20 °C] | 0,1017 |
| CCP-3OCF₂.F.F | 15,0 % | Δε [1 kHz, 20 °C] | |
| CCP-5OCF₂.F.F | 16,0 % | V_{(10,0,20)} [V] | 1,24 |
| CUP-2F.F | 6,0 % | V_{(90,0,20)} [V] | 2,01 |
| CUP-3F.F | 6,0 % | ν 20 | 50 cSt |
| CUP-5F.F | 5,0 % | ν -30 | 4630 cSt |
| CUP-2OCF₂.F.F | 6,0 % | HR (100°) | 91 % |
| CUP-3OCF₂.F.F | 6,0 % | | |
| CUP-5OCF₂.F.F | 6,0 % | | |
| CBC-33F | 3,5 % | | |
| CBC-53F | 4,0 % | | |
| CBC-55F | 4,0 % | | |

| Beispiel 6 | | | |
|---|---|---|---|
| PCH-5F | 3,0 % | S → N [°C] | |
| CCP-2OCF₂.F.F | 16,0 % | Klärpunkt [°C] | +109 |
| CCP-3OCF₂.F.F | 15,0 % | Δn [589 nm, 20 °C] | +0,0998 |
| CCP-5CCF₂.F.F | 16,0 % | Δε [1 kHz, 20 °C] | |
| CUP-2F.F | 6,0 % | V_{(10,0,20)} [V] | 1,27 |
| CUP-3F.F | 6,0 % | V_{(90,0,20)} [V] | 2,01 |
| CUP-5F.F | 5,0 % | | |
| CUP-2OCF₂.F.F | 6,0 % | | |
| CUP- 3OCF₂.F.F | 6,0 % | | |
| CUP-5OCF₂.F.F | 6,0 % | | |
| CBC-33F | 5,0 % | | |
| CBC-34F | 5,0 % | | |
| CBC-35F | 5,0 % | | |

| Beispiel 7 | | | |
|---|---|---|---|
| PCH-5F | 3,0 % | S → N [°C] | |
| CCP-2OCF₂.F.F | 16,0 % | Klärpunkt [°C ] | +97 |
| CCP-3OCF₂.F.F | 15,0 % | Δn [589 nm, 20 °C] | +0,1050 |
| CCP-SOCF₂.F.F | 16,0 % | Δε [1 kHz, 20 °C] | |
| CUP-2F.F | 5,0 % | V_{(10,0,20)} [V] | 1,15 |
| CUP-3F.F | 5,0 % | V_{(90,0,20)} [V] | 1,82 |
| CUP-5F.F | 5,0 % | | |
| CUP-3OCF₃.F.F | 10,0 % | | |
| CUP-5OCF₃.F.F | 10,0 % | | |
| CBC-33F | 5,0 % | | |
| CBC-53F | 5,0 % | | |
| CBC-55F | 5,0 % | | |

| Beispiel 8 | | | |
|---|---|---|---|
| PCH-5F | 3,0 % | S → N [°C] | |
| CCP-2OCF₂.F.F | 16,0 % | Klärpunkt [°C] | +103 |
| CCP-3OCF₂.F.F | 15,0 % | Δn [589 nm, 20 °C] | +0,1075 |
| CCP-SOCF₂.F.F | 16,0 % | Δε [1 kHz, 20 °C] | |
| CUP-2F.F | 5,0 % | V_{(10,0,20)} [V] | 1,27 |
| CUP-3F.F | 5,0 % | V_{(90,0,20)} [V] | 2,02 |
| CUP-5F.F | 5,0 % | | |
| CUP-3OCF₃.F.F | 10,0 % | | |
| CUP-5OCF₃.F.F | 10,0 % | | |
| CBC-33F | 5,0 % | | |
| CBC-53F | 5,0 % | | |
| CBC-55F | 5,0 % | | |

| Beispiel 9 | | | |
|---|---|---|---|
| PCH-5F | 3,0 % | S → N [°C] | |
| CCP-2OCF₂.F.F | 16,0 % | Klärpunkt [°C] | +100 |
| CCP-3OCF₂.F.F | 15,0 % | Δn [589 nm, 20 °C] | +0,1068 |
| CCP-SOCF₂.F.F | 16,0 % | Δε [1 kHz, 20 °C] | |
| CUP-2F.F | 6,0 % | V_{(10,0,20)} [V] | 1,27 |
| CUP-3F.F | 6,0 % | V_{(90,0,20)} [V] | 1,99 |
| CUP-5F.F | 5,0 % | | |
| CUP-2OCF₃.F.F | 6,0 % | | |
| CUP-3OCF₃.F.F | 6,0 % | | |
| CUP-5OCF₃.F.F | 6,0 % | | |
| CBC-33F | 5,0 % | | |
| CBC-53F | 5,0 % | | |
| CBC-55F | 5,0 % | | |

| Beispiel 10 | | | |
|---|---|---|---|
| PCH-6F | 4,0 % | S → N [°C] | |
| PCH-7F | 4,0 % | Klärpunkt [°C] | +77 |
| CCP-2OCF₂.F.F | 16,0 % | Δn [589 nm, 20 °C] | 0,0980 |
| CCP-3OCF₂.F.F | 15,0 % | Δε [1 kHz, 20 °C] | |
| CCP-5OCF₂.F.F | 16,0 % | V_{(10,0,20)} [V] | |
| CUP-2F.F | 6,0 % | V_{(90,0,20)} [V] | |
| CUP-3F.F | 6,0 % | | |
| CUP-5F.F | 5,0 % | | |
| CUP-2OCF₃.F.F | 6,0 % | | |
| CUP-3OCF₃.F.F | 6,0 % | | |
| CUP-5OCF₃.F.F | 6,0 % | | |
| CBC-33F | 4,0 % | | |
| CBC-53F | 3,0 % | | |
| CBC-55F | 3,0 % | | |

| Beispiel 11 | | | |
|---|---|---|---|
| PCH-6F | 6,0 % | S → N [°C] | |
| CCP-2OCF₂.F.F | 16,0 % | Klärpunkt [°C ] | +85 |
| CCP-3OCF₂.F.F | 15,0 % | Δn [589 nm, 20 °C] | +0,1015 |
| CCP-5CCF₂.F.F | 16,0 % | Δε [1 kHz, 20 °C] | |
| CUP-2F.F | 6,0 % | V_{(10,0,20)} [V] | 1,16 |
| CUP-3F.F | 6,0 % | V_{(90,0,20)} [V] | 1,83 |
| CUP-5F.F | 5,0 % | | |
| CUP-2OCF₃.F.F | 6,0 % | | |
| CUP-3OCF₃.F.F | 6,0 % | | |
| CUP-5OCF₃.F.F | 6,0 % | | |
| CBC-33F | 4,0 % | | |
| CBC-53F | 4,0 % | | |
| CBC-55F | 4,0 % | | |

| Beispiel 12 | | | |
|---|---|---|---|
| PCH-6F | 3,5 % | S → N [°C] | |
| PCH-7F | 3,0 % | Klärpunkt [°C] | |
| CCP-2OCF₂.F.F | 12,0 % | Δn [589 nm, 20 °C] | |
| CCP-3OCF₂.F.F | 12,0 % | Δε [1 kHz, 20 °C] | |
| CCP-5OCF₂.F.F | 12,0 % | V_{(10,0,20)} [V] | |
| CUP-2F.F | 5,0 % | V_{(90,0,20)} [V] | |
| CUP-3F.F | 5,0 % | | |
| CUP-5F.F | 5,0 % | | |
| CUP-2OCF₃.F.F | 5,0 % | | |
| CUP-3OCF₃.F.F | 5,0 % | | |
| CUP-5OCF₃.F.F | 5,0 % | | |
| CUP-3OCCF₂.F.F | 8,0 % | | |
| CUP-5OCCF₂.F.F | 8,0 % | | |
| CBC-33F | 3,5 % | | |
| CBC-53F | 4,0 % | | |
| CBC-55F | 4,0 % | | |

| Beispiel 13 | | | |
|---|---|---|---|
| PCH-5F | 5,0 % | S → N [°C] | |
| PCH-7F | 6,0 % | Klärpunkt [°C] | +83 |
| CCP-2OCF₃ | 11,0 % | Δn [589 nm, 20 °C] | +0,0882 |
| CCP-3OCF₃ | 12,0 % | Δε [1 kHz, 20 °C] | |
| CCP-4OCF₃ | 10,0 % | V_{(10,0,20)} [V] | 1,40 |
| CCP-5OCF₃ | 12,0 % | V_{(90,0,20)} [V] | 2,18 |
| CUP-3OCF₃.F.F | 12,0 % | | |
| BCH-5F.F.F | 11,0 % | | |
| CCP-3F.F.F | 12,0 % | | |
| CCP-5F.F.F | 9,0 % | | |

| Beispiel 14 | |
|---|---|
| PCH-5F | 9,0 % |
| PCH-6F | 7,2 % |
| PCH-7F | 5,4 % |
| CCP-2OCF₃ | 7,2 % |
| CCP-3OCF₃ | 10,8 % |
| CCP-4OCF₃ | 6,3 % |
| CCP-5OCF₃ | 9,9 % |
| BCH-3F.F | 10,8 % |
| BCH-5F.F | 9,0 % |
| ECCP-3OCF₃ | 4,5 % |
| ECCP-5OCF₃ | 4,5 % |
| CBC-33F | 1,8 % |
| CBC-53F | 1,8 % |
| CBC-55F | 1,8 % |
| CUP-3OCF₃.F.F | 10,0 % |

| Beispiel 15 | | | |
|---|---|---|---|
| PCH-5F | 5,0 % | S → N [°C] | < -20 |
| PCH-7F | 6,0 % | Klärpunkt [°C] | +80 |
| CCP-2OCF₃ | 11,0 % | Δn [589 nm, 20 °C] | +0,0858 |
| CCP-3OCF₃ | 12,0 % | V_{(10,0,20)} [V] | 1,36 |
| CCP-4OCF₃ | 10,0 % | V_{(90,0,20)} [V] | 2,15 |
| CCP-5OCF₃ | 12,0 % | | |
| CUP-3OCF₃.F.F | 12,0 % | | |
| CUP-5OCF₃.F.F | 11,0 % | | |
| CCP-3F.F.F | 12,0 % | | |
| CCP-3F.F.F | 9,0 % | | |

| Beispiel 16 | | | |
|---|---|---|---|
| PCH-5F | 3,0 % | S → N [°C] | < -30 |
| CCP-2OCF₂.F.F | 16,0 % | Klärpunkt [°C] | +101 |
| CCP-3OCF₂.F.F | 15,0 % | Δn [589 nm, 20 °C] | +0,1071 |
| CCP-5OCF₂.F.F | 16,0 % | V_{(10,0,20)} [V] | 1,23 |
| CUP-2F.F | 5,0 % | V_{(90,0,20)} [V] | 1,94 |
| CUP-3F.F | 5,0 % | | |
| CUP-5F.F | 5,0 % | | |
| CUP-3OCF₃.F.F | 10,0 % | | |
| CUP-5OCF₃.F.F | 10,0 % | | |
| CBC-33F | 5,0 % | | |
| CBC-53F | 5,0 % | | |
| CBC-55F | 5,0 % | | |

| Beispiel 17 | |
|---|---|
| PCH-5F | 8,5 % |
| PCH-6F | 6,8 % |
| PCH-7F | 5,1 % |
| CCP-2OCF₃ | 6,8 % |
| CCP-3OCF₃ | 10,2 % |
| CCP-4OCF₃ | 6,0 % |
| CCP-5OCF₃ | 9,3 % |
| BCH-3F.F | 10,2 % |
| BCH-5F.F | 8,5 % |
| ECCP-3OCF₃ | 4,2 % |
| ECCP-5OCF₃ | 4,2 % |
| CBC-33F | 1,7 % |
| CBC-53F | 1,7 % |
| CBC-55F | 1,7 % |
| CUP-3OCF₃.F.F | 15,0 % |

| Beispiel 18 | | | |
|---|---|---|---|
| PCH-5F | 8,0 % | Klärpunkt [°C] | 75,6 |
| PCH-6F | 6,4 % | | |
| PCH-7F | 4,8 % | | |
| CCP-2OCF₃ | 6,4 % | | |
| CCP-3OCF₃ | 9,6 % | | |
| CCP-4OCF₃ | 5,6 % | | |
| CCP-5OCF₃ | 8,8 % | | |
| BCH-3F.F | 9,6 % | | |
| BCH-5F.F | 8,0 % | | |
| ECCP-3OCF₃ | 4,0 % | | |
| ECCP-5OCF₃ | 4,0 % | | |
| CBC-33F | 1,6 % | | |
| CBC-53F | 1,6 % | | |
| CBC-55F | 1,6 % | | |
| CUP-3OCF₃.F.F | 20,0 % | | |

| Beispiel 19 | | | |
|---|---|---|---|
| PCH-6F | 1,0 % | Klärpunkt [°C] | +74 |
| CCP-2OCF₂.F.F | 17,0 % | Δn [589 nm, 20 °C] | +0,0994 |
| CCP-3OCF₂.F.F | 17,0 % | V_{(10,0,20)} [V] | 1,00 |
| CCP-5OCF₂.F.F | 17,0 % | V_{(90,0,20)} [V] | 1,59 |
| CUP-2F.F | 7,0 % | | |
| CUP-3F.F | 7,0 % | | |
| CUP-5F.F | 8,0 % | | |
| CUP-3OCF₃.F.F | 11,0 % | | |
| CUP-5OCF₃.F.F | 11,0 % | | |
| CBC-53F | 2,0 % | | |
| CBC-55F | 2,0 % | | |

| Beispiel 20 | | | |
|---|---|---|---|
| PCH-6F | 1,0 % | Klärpunkt [°C ] | +83 |
| CCP-2OCF₂.F.F | 16,0 % | Δn [589 nm, 20 °C] | +0,1025 |
| CCP-3OCF₂.F.F | 17,0 % | V_{(10,0,20)} [V] | 1,11 |
| CCP-5OCF₂.F.F | 17,0 % | V_{(90,0,20)} [V] | 1,80 |
| CUP-2F.F | 6,0 % | | |
| CUP-3F.F | 7,0 % | | |
| CUP-5F.F | 8,0 % | | |
| CUP-3OCF₃.F.F | 10,0 % | | |
| CUP-5OCF₃.F.F | 11,0 % | | |
| CBC-53F | 3,0 % | | |
| CBC-55F | 4,0 % | | |

| Beispiel 21 | | | |
|---|---|---|---|
| CCP-2OCF₂.F.F | 16,0 % | Klärpunkt [°C] | 86 |
| CCP-3OCF₂.F.F | 16,0 % | Δn [589 nm, 20 °C] | +0,1046 |
| CCP-5OCF₂.F.F | 17,0 % | V_{(10,0,20)} [V] | 1,10 |
| CUP-2F.F | 6,0 % | V_{(90,0,20)} [V] | 1,76 |
| CUP-3F.F | 7,0 % | | |
| CUP-5F.F | 8,0 % | | |
| CUP-3OCF₃.F.F | 11,0 % | | |
| CUP- 5OCF₃.F.F | 11,0 % | | |
| CBC-53F | 4,0 % | | |
| CBC-55F | 4,0 % | | |

| Beispiel 22 | | | |
|---|---|---|---|
| PCH-5F | 9,0 % | Klärpunkt [°C] | 80,6 |
| PCH-6F | 7,2 % | Δn [589 nm, 20 °C] | 0,0954 |
| PCH-7F | 5,4 % | Δε [1 kHz, 20 °C] | 6,67 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-1OCF₃.F.F | 10,0 % | | |

| Beispiel 23 | | | |
|---|---|---|---|
| PCH-5F | 9,0 % | Klärpunkt [°C] | 81,8 |
| PCH-6F | 7,2 % | Δn [589 nm, 20 °C] | 0,0956 |
| PCH-7F | 5,4 % | Δε [1 kHz, 20 °C] | 6,59 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BZH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-2OCF₃.F.F | 10,0 % | | |

| Beispiel 24 | | | |
|---|---|---|---|
| PCH-5F | 9,0 % | Klärpunkt [°C] | 84,5 |
| PCH-6F | 7,2 % | Δn [589 nm, 20 °C] | 0,0974 |
| PCH-7F | 5,4 % | Δε [1 kHz, 20 °C] | 6,68 |
| CCP- 2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-3OCF₃.F.F | 10,0 % | | |

| Beispiel 25 | | | |
|---|---|---|---|
| PCH-5F | 9,0 % | Klärpunkt [°C] | 85,2 |
| PCH-6F | 7,2 % % | Δn [589 nm, 20 °C] | 0,0970 |
| PCH-7F | 5,4 % | Δε [1 kHz, 20 °C] | 6,60 |
| CCP-2OCF₃ | 7,2 % | | |
| CCP-3COF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-5OCF₃.F.F | 10,0 % | | |

Δ

| Beispiel 26 | | | |
|---|---|---|---|
| PCH-5F | 5, 0 % | Klrpunkt [°C] | +81 |
| PCH-7F | 6,0 % | Δn [589 nm, 20 °C] | 0,0884 |
| CCP-2OCF₃ | 11,0 % | V_{(10,0,20)} [V] | 1,42 |
| CCP-3OCF₃ | 12,0 % | V_{(90,0,20)} [V] | 2,22 |
| CCP-4OCF₃ | 10,0 % | | |
| CCP-5OCF₃ | 12,0 % | | |
| CUP-2OCF₂.F.F | 12,0 % | | |
| BCH-5F.F.F | 11,0 % | | |
| CCP-3F.F.F | 12,0 % | | |
| CCP-5F.F.F | 9,0 % | | |

| Beispiel 27 | | | |
|---|---|---|---|
| PCH-5F | 5,0 % | Klärpunkt [°C] | +84 |
| PCH-7F | 6,0 % | Δn [589 nm, 20 °C] | 0,0903 |
| CCP-2OCF₃ | 11,0 % | V_{(10,0,20)} [V] | 1,45 |
| CCP-3OCF₃ | 12,0 % | V_{(90,0,20)} [V] | 2,30 |
| CCP-4OCF₃ | 10,0 % | | |
| CCP-5OCF₃ | 12,0 % | | |
| CUP-3OCF₂.F.F | 12,0 % | | |
| BCH-5F.F.F | 11,0 % | | |
| CCP-3F.F.F | 12,0 % | | |
| CCP-5F.F.F | 9,0 % | | |

| Beispiel 28 | | | |
|---|---|---|---|
| PCH-5F | 5,0 % | Klärpunkt [°C] | +83 |
| PCH-7F | 6,0 % | Δn [589 nm, 20 °C] | 0,0908 |
| CCP-2OCF₃ | 11,0 % | V_{(10,0,20)} [V] | 1,46 |
| CCP-3OCF₃ | 12,0 % | V_{(90,0,20)} [V] | 2,27 |
| CCP-4OCF₃ | 10,0 % | | |
| CCP-5OCF₃ | 12,0 % | | |
| BCH-3F.F.F | 12,0 % | | |
| CUP-5OCF₂.F.F | 11,0 % | | |
| CCP-3F.F.F | 12,0 % | | |
| CCP-5F.F.F | 9,0 % | | |

| Beispiel 29 | | | |
|---|---|---|---|
| PCH-5F | 5,0 % | Klärpunkt [°C] | +81 |
| PCH-7F | 6,0 % | Δn [589 nm, 20 °C] | 0,0884 |
| CCP-2OCF₃ | 11,0 % | V_{(10,0,20)} [V] | 1,23 |
| CCP-3OCF₃ | 12,0 % | V_{(90,0,20)} [V] | 1,95 |
| CCP-4OCF₃ | 10,0 % | | |
| CCP-5OCF₃ | 12,0 % | | |
| CUP-2OCF₂.F.F | 12,0 % | | |
| BCH-5F.F.F | 11,0 % | | |
| CCP-3F.F.F | 12,0 % | | |
| CCP-5F.F.F | 9,0 % | | |

| Beispiel 30 | | | |
|---|---|---|---|
| PCH-6F | 5,0 % | Klärpunkt [°C] | +85 |
| CCP-2OCF₂.F.F | 19,0 % | Δn [589 nm, 20 °C] | 0,0993 |
| CCP-3OCF₂.F.F | 19,0 % | V_{(10,0,20)} [V] | 1,23 |
| CCP-5OCF₂.F.F | 19,0 % | V_{(90,0,20)} [V] | 1,89 |
| CUP-2F.F | 5,0 % | | |
| CUP-3F.F | 5,0 % | | |
| CUP-5F.F | 5,0 % | | |
| CUP-20CF₂.F.F | 5,0 % | | |
| CUP-30CF2.F.F | 5,0 % | | |
| CUP-50CF₂.F.F | 5,0 % | | |
| CBC-33F | 4,0 % | | |
| CBC-53F | 4,0 % | | |

| Beispiel 31 | | | |
|---|---|---|---|
| PCH-6F | 3,5 % | Klärpunkt [°C] | +92 |
| PCH-7F | 3,0 % | Δn [589 nm, 20 °C] | +0,0942 |
| CCP-2OCF₂.F.F | 16,0 % | V_{(10,0,20)} [V] | 1,25 |
| CCP-3OCF₂.F.F | 15,0 % | V_{(90,0,20)} [V] | 1,94 |
| CCP-5OCF₂.F.F | 16,0 % | | |
| CUP-2F.F | 6,0 % | | |
| CUP-3F.F | 6,0 % | | |
| CUP-5F.F | 5,0 % | | |
| CUP-20CF₂.F.F | 6,0 % | | |
| CUP-30CF₂.F.F | 6,0 % | | |
| CUP-50CF₂.F.F | 6,0 % | | |
| CCPC-33 | 3,5 % | | |
| CCPC-34 | 4,0 % | | |
| CCPC-35 | 4,0 % | | |

| Beispiel 32 | | | |
|---|---|---|---|
| PCH-6F | 4,5 % % | Klärpunkt [°C] | +86 |
| PCH-7F | 3,0 % | Δn [589 nm, 20 °C] | +0,0952 |
| CCP-2OCF₂.F.F | 16,0 % | V_{(10,0,20)} [V] | 1,21 |
| CCP-3OCF₂.F.F | 15,0 % | V_{(90,0,20)} [V] | 1,88 |
| CCP-5OCF₂.F.F | 16,0 % | | |
| CUP-2F.F | 6,0 % | | |
| CUP-3F.F | 6,0 % | | |
| CUP-5F.F | 5,0 % | | |
| CUP-20CF₂.F.F | 6,0 % | | |
| CUP-30CF₂.F.F | 6,0 % | | |
| CUP-50CF₂.F.F | 6,0 % | | |
| CCPC-33 | 2,5 % | | |
| CCPC-34 | 4,0 % | | |
| CCPC-35 | 4,0 % | | |

| Beispiel 33 | | | |
|---|---|---|---|
| PCH-SF | 4,5 % | Klärpunkt [°C] | +101 |
| CCP-2OCF₂.F.F | 16,0 % | Δn [589 nm, 20 °C] | +0,0984 |
| CCP-3OCF₂.F.F | 15,0 % | V_{(10,0,20)} [V] | 1,22 |
| CCP-5OCF₂.F.F | 16,0 % | V_{(90,0,20)} [V] | 1,92 |
| CUP-2F.F | 6,0 % | | |
| CUP-3F.F | 6,0 % | | |
| CUP-5F.F | 5,0 % | | |
| CUP-20CF₂.F.F | 6,0 % | | |
| CUP-30CF₂.F.F | 6,0 % | | |
| CUP-50CF₂.F.F | 6,0 % | | |
| CCPC-33 | 4,0 % | | |
| CCPC-34 | 4,5 % | | |
| CCPC-35 | 5,0 % | | |

| Beispiel 34 | | | |
|---|---|---|---|
| CCP-2OCF₂.F.F | 16,0 % | Klärpunkt [°C] | 96 |
| CCP-3OCF₂.F.F | 15,0 % | Δn [589 nm, 20 °C] | +0,1010 |
| CCP-5OCF₂.F.F | 16,0 % | V_{(10,0,20)} [V] | 1,27 |
| CUP-2F.F | 6,0 % | V_{(90,0,20)} [V] | 1,94 |
| CUP-3F.F | 6,0 % | | |
| CUP-5F.F | 5,0 % | | |
| CUP-20CF₂.F.F | 6,0 % | | |
| CUP-30CF₂.F.F | 6,0 % | | |
| CUP-50CF₂.F.F | 6,0 % | | |
| CBC-33F | 6,0 % | | |
| CBC-53F | 6,0 % | | |
| CBC-55F | 6,0 % | | |

| Beispiel 35 | | | |
|---|---|---|---|
| PCH-6F | 3,5 % | Klärpunkt [°C] | 96 |
| PCH-7F - | 8,0 % | Δn [589 nm, 20 °C] | +0,1010 |
| CCP-20CF₂.F.F | 16,0 % | V_{(10,0,20)} [V] | 1,38 |
| CCP-30CF₂.F.F | 15,0 % | V_{(90,0,20)} [V] | 2,10 |
| CCP-50CF₂.F.F | 16,0 % | | |
| CUP-2F.F | 6,0 % | | |
| CUP-3F.F | 6,0 % | | |
| CUP-5F.F | 5,0 % | | |
| CUP-20CF₂.F.F | 6,0 % | | |
| CUP-30CF₂.F.F | 6,0 % | | |
| CUP-50CF₂.F.F | 6,0 % | | |
| CBC-33F | 3,5 % | | |
| CBC-53F | 4,0 % | | |
| CBC-55F | 4,0 % | | |

| Beispiel 36 | | | |
|---|---|---|---|
| PCH-5F | 8,5 % | Klärpunkt [°C] | 80 |
| PCH-6F | 6,8 % | | |
| PCH-7F | 5,1 % | | |
| CCP-20CF₃ | 6,8 % | | |
| CCP-30CF₃ | 10,2 % | | |
| CCP-40CF₂ | 6,0 % | | |
| CCP-50CF₃ | 9,3 % | | |
| BCH-3F.F | 10,2 % | | |
| BCH-5F.F | 8,5 % | | |
| ECCP-3OCF₃ | 4,2 % | | |
| ECCP-5OCF₃ | 4,2 % | | |
| CBC-33F | 1,7 % | | |
| CBC-53F | 1,7 % | | |
| CBC-55F | 1,7 % | | |
| CUP-3OCF₂.F.F | 15,0 % | | |

| Beispiel 37 | | | |
|---|---|---|---|
| PCH-7F | 1,0 % | Klärpunkt [°C] | +84 |
| CCP-2OCF₂.F.F | 16,0 % | Δn [589 nm, 20 °C] | +0,1050 |
| CCP-3OCF₂.F.F | 16,0 % | V_{(10,0,20)} [V] | 1,09 |
| CCP-5OCF₂.F.F | 16,0 % | V_{(90,0,20)} [V] | 1,74 |
| CUP-2F.F | 5,0 % | | |
| CUP-3F.F | 5,0 % | | |
| CUP-5F.F | 5,0 % | | |
| CUP-20CF₂.F.F | 5,0 % | | |
| CUP-30CF₂.F.F | 5,0 % | | |
| CUP-50CF₂.F.F | 5,0 % | | |
| CUP-20CCF₂.F.F | 5,0 % | | |
| CUP-30CCF₂.F.F | 5,0 % | | |
| CBC-53F | 3,0 % | | |
| CBC-55F | 3,0 % | | |

| Beispiel 38 | | | |
|---|---|---|---|
| PCH-5F | 9,0 % | Klärpunkt [°C] | +81,6 |
| PCH-6F | 7,2 % | Δn [589 nm, 20 °C] | 0,0965 |
| PCH-7F | 5,4 % | Δε [1 kHz, 20 °C] | 6,17 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-20CF₂.F.F | 10,0 % | | |

| Beispiel 39 | | | |
|---|---|---|---|
| PCH-5F | 9,0 % | Klärpunkt [°C] | 84,0 |
| PCH-6F | 7,2 % | Δn [589 nm, 20 °C] | 0,0980 |
| PCH-7F | 5,4 % | Δε [1 kHz, 20 °C] | 6,18 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-30CF₂.F.F | 10,0 % | | |

| Beispiel 40 | | | |
|---|---|---|---|
| PCH-5F | 9,0 % | Klärpunkt [°C] | 84,7 |
| PCH-6F | 7,2 % | Δn [589 nm, 20 °C] | 0,0975 |
| PCH-7F | 5,4 % | Δε [1 kHz, 20 °C] | 6,21 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-50CF₂.F.F | 10,0 % | | |

| Beispiel 41 | | | |
|---|---|---|---|
| PCH-6F | 4,5 % | S → N [°C] | < -40 |
| PCH-7F | 4,0 % | Klärpunkt [°C] | +84 |
| CCP-2OCF₂.F.F | 16,0 % | Δn [589 nm, 20 °C] | +0,1012 |
| CCP-3OCF₂.F.F | 15,0 % | V_{(10,0,20)} [V] | 1,17 |
| CCP-5OCF₂.F.F | 16,0 % | V_{(50,0,20)} [V] | 1,47 |
| CUP-2F.F | 6,0 % | V_{(90,0,20)} [V] | 1,87 |
| CUP-3F.F | 6,0 % | ν 20: | 44 cSt |
| CUP-5F.F | 5,0 % | ν -40: | 19000 cSt |
| CUP-3OCCF₂.F.F | 9,0 % | HR (100 °C) : | 92 % |
| CUP-5OCCF₂.F.F | 9,0 % | | |
| CBC-33F | 3,5 % | | |
| CBC-53F | 3,5 % | | |
| CBC-55F | 3,0 % | | |

| Beispiel 42 | | | |
|---|---|---|---|
| PCH-5F | 3,0 % | S → N [°C] | |
| CCP-2OCF₂.F.F | 16,0 % | Klärpunkt [°C] | +109 |
| CCP-3OCF₂.F.F | 15,0 % | Δn [589 nm, 20 °C] | +0,1111 |
| CCP-5OCF₂.F.F | 16,0 % | Δε [1 kHz, 20 °C] | |
| CUP-2F.F | 5,0 % | V_{(10,0,20)} [V] | 1,29 |
| CUP-3F.F | 5,0 % | V_{(90,0,20)} [V] | 2,03 |
| CUP-5F.F | 5,0 % | | |
| CUP-3OCCF₂.F.F | 10,0 % | | |
| CUP-5OCCF₂.F.F | 10,0 % | | |
| CBC-33F | 5,0 % | | |
| CBC-53F | 5,0 % | | |
| CBC-55F | 5,0 % | | |

| Beispiel 43 | | | |
|---|---|---|---|
| PCH-5F | 6,0 % | S → N [°C] | |
| CCP-2OCF₂.F.F | 16,0 % | Klärpunkt [°C] | +96 |
| CCP-3OCF₂.F.F | 15,0 % | Δn [589 nm, 20 °C] | +0,1061 |
| CCP-5OCF₂.F.F | 16,0 % | Δε [1 kHz, 20 °C] | |
| CUP-2F.F | 5,0 % | V_{(10,0,20)} [V] | 1,24 |
| CUP-3F.F | 5,0 % | V_{(90,0,20)} [V] | 1,92 |
| CUP-5F.F | 5,0 % | | |
| CUP-3OCCF₂.F.F | 10,0 % | | |
| CUP-5OCCF₂.F.F | 10,0 % | | |
| CBC-33F | 4,0 % | | |
| CBC-53F | 4,0 % | | |
| CBC-55F | 4,0 % | | |

| Beispiel 44 | | | |
|---|---|---|---|
| PCH-5F | 5,0 % | S → N [°C] | |
| CCP-2OCF₂.F.F | 16,0 % | Klärpunkt [°C] | +100 |
| CCP-3OCF₂.F.F | 15,0 % | Δn [589 nm, 20 °C] | +0,1077 |
| CCP-5OCF₂.F.F | 16,0 % | Δε [1 kHz, 20 °C] | |
| CUP-2F.F | 5,0 % | V_{(10,0,20)} [V] | 1,25 |
| CUP-3F.F | 5,0 % | V_{(90.0.20)} [V] | 1,97 |
| CUP-5F.F | 5,0 % | | |
| CUP-3OCCF₂.F.F | 10,0 % | | |
| CUP-5OCCF₂.F.F | 10,0 % | | |
| CBC-33F | 5,0 % | | |
| CBC-53F | 4,5 % | | |
| CBC-55F | 3,5 % | | |

| Beispiel 45 | | | |
|---|---|---|---|
| PCH-6F | 3,5 % | S → N [°C] | |
| PCH-7F | 3,0 % | Klärpunkt [°C] | +88 |
| CCP-2OCF₂.F.F | 16,0 % % | Δn [589 nm, 20 °C] | +0,1036 |
| CCP-3OCF₂.F.F | 15,0 % % | Δε [1 kHz, 20 °C] | |
| CCP-5OCF₂.F.F | 16,0 % | V_{(10,0,20)} [V] | 1,14 |
| CUP-2F.F | 6,0 % | V_{(90,0,20)} [V] | 1,79 |
| CUP-3F.F | 6,0 % | | |
| CUP-5F.F | 5,0 % | | |
| CUP-3OCCF₂.F.F | 10,0 % | | |
| CUP-5OCCF₂.F.F | 10,0 % | | |
| CBC-33F | 3,5 % | | |
| CBC-53F | 3,0 % | | |
| CBC-55F | 3,0 % | | |

| Beispiel 46 | | | |
|---|---|---|---|
| PCH-6F | 5,5 % | S → N [°C] | |
| PCH-7F | 5,0 % | Klärpunkt [°C] | +81 |
| CCP-2OCF₂.F.F | 16,0 % | Δn [589 nm, 20 °C] | +0,0994 |
| CCP-3OCF₂.F.F | 15,0 % | Δε [1 kHz, 20 °C] | |
| CCP-5OCF₂.F.F | 16,0 % | V_{(10,0,20)} [V] | 1,21 |
| CUP-2F.F | 5,0 % | V_{(90,0,20)} [V] | 1,88 |
| CUP-3F.F | 5,0 % | | |
| CUP-5F.F | 5,0 % | | |
| CUP-3OCCF₂.F.F | 9,0 % | | |
| CUP-5OCCF₂.F.F | 9,0 % | | |
| CBC-33F | 3,5 % | | |
| CBC-53F | 3,0 % | | |
| CBC-55F | 3,0 % | | |

| Beispiel 47 | | | |
|---|---|---|---|
| PCH-6F | 4,5 % | S → N [°C] | < -40 |
| PCH-7F | 4,0 % | Klärpunkt [°C ] | +84 |
| CCP-2OCF₂.F.F | 16,0 % | Δn [589 nm, 20 °C] | +0,1012 |
| CCP-3OCF₂.F.F | 15,0 % | Δε [1 kHz, 20 °C] | |
| CCP-5OCF₂.F.F | 16,0 % | V_{(10,0,20)} [V] | 1,18 |
| CUP-2F.F | 6,0 % | V_{(90,0,20)} [V] | 1,87 |
| CUP-3F.F | 6,0 % | | |
| CUP-5F.F | 5,0 % | | |
| CUP-3OCCF₂.F.F | 9,0 % | | |
| CUP-5OCCF₂.F.F | 9,0 % | | |
| CBC-33F | 3,5 % | | |
| CBC-53F | 3,0 % | | |
| CBC-55F | 3,0 % | | |

| Beispiel 48 | | | |
|---|---|---|---|
| PCH-6F | 4,5 % | S → N [°C] | < -40 |
| PCH-7F | 4,0 % | Klärpunkt [°C] | +93 |
| CCP-2OCF₂.F.F | 8,0 % | Δn [589 nm, 20 °C] | +0,1036 |
| CCP-3OCF₂.F.F | 8,0 % | Δε [1 kHz, 20 °C] | |
| CCP-5OCF₂.F.F | 8,0 % | V_{(10,0,20)} [V] | 1,21 |
| CCP-2OCCF₂.F.F | 8,0 % | V_{(90,0,20)} [V] | 1,92 |
| CCP-3OCCF₂.F.F | 7,0 % | | |
| CCP-5OCCF₂.F.F | 8,0 % | | |
| CUP-2F.F | 6,0 % | | |
| CUP-3F.F | 6,0 % | | |
| CUP-5F.F | 5,0 % | | |
| CUP-3OCCF₂.F.F | 9,0 % | | |
| CUP-5OCCF₂.F.F | 9,0 % | | |
| CBC-33F | 3,5 % | | |
| CBC-53F | 3,0 % | | |
| CBC-55F- | 3,0 % | | |

| Beispiel 49 | | | |
|---|---|---|---|
| PCH-6F | 4,5 % | S → N [°C] | < -40 |
| PCH-7F | 4,0 % | Klärpunkt [°C] | +96 |
| CCP-2OCCF₂.F.F | 16,0 % | Δn [589 nm, 20 °C] | +0,108 |
| CCP-3OCCF₂.F.F | 15,0 % | Δε [1 kHz, 20 °C] | |
| CCP-5OCCF₂.F.F | 16,0 % | V_{(10,0,20)} [V] | 1,23 |
| CUP-2F.F | 6,0 % | V_{(90,0,20)} [V] | 1,95 |
| CUP-3F.F | 6,0 % | | |
| CUP-5F.F | 5,0 % | | |
| CUP-3OCCF₂.F.F | 9,0 % | | |
| CUP-5OCCF₂.F.F | 9,0 % | | |
| CBC-33F | 3,5 % | | |
| CBC-53F | 3,0 % | | |
| CBC-55F | 3,0 % | | |

| Beispiel 50 | | | |
|---|---|---|---|
| PCH-6F | 4,5 % | S → N [°C] | < -40 |
| PCH-7F | 4,0 % | Klärpunkt [°C] | +87 |
| CCP-2OCF₂.F.F | 16,0 % | Δn [589 nm, 20 °C] | +0,103 |
| CCP-3OCF₂.F.F | 15,0 % | Δε [1 kHz, 20 °C] | |
| CCP-5OCF₂.F.F | 16,0 % | V_{(10,0,20)} [V] | 1,15 |
| CUP-2OCH=CF₂.F.F | 6,0 % | V_{(90,0,20)} [V] | 1,86 |
| CUP-3OCH=CF₂.F.F | 6,0 % | | |
| CUP-5OCH=CF₂.F.F | 5,0 % | | |
| CUP-3OCCF₂.F.F | 9,0 % | | |
| CUP-5OCCF₂.F.F | 9,0 % | | |
| CBC-33F | 3,5 % | | |
| CBC-53F | 3,0 % | | |
| CBC-55F | 3,0 % | | |

| Beispiel 51 | | | |
|---|---|---|---|
| PCH-6F | 4,5 % | S → N [°C ] | < -40 |
| PCH-7F | 4,0 % | Klärpunkt [°C] | 93 |
| CCP-2OCF=CF₂.F.F | 16,0 % | Δn [589 nm, 20 °C] | +0,105 |
| CCP-3OCF=CF₂.F.F | 15,0 % | Δε [1 kHz, 20 °C] | |
| CCP-5OCF=CF₂.F.F | 16,0 % | V_{(10,0,20)} [V] | 1,13 |
| CUP-2F.F | 6,0 % | V_{(90,0,20)} [V] | 1,80 |
| CUP-3F.F | 6,0 % | | |
| CUP-5F.F | 5,0 % | | |
| CUP-3OCCF₂.F.F | 9,0 % | | |
| CUP-5OCCF₂.F.F | 9,0 % | | |
| CBC-33F | 3,5 % | | |
| CBC-53F | 3,5 % | | |
| CBC-55F | 3,0 % | | |

| Beispiel 52 | | | |
|---|---|---|---|
| PCH-5F | 3,0 % | S → N [°C] | |
| CCP-2OCF₂.F.F | 16,0 % | Klärpunkt [°C] | +96 |
| CCP-3OCF₂.F.F | 15,0 % | Δn [589 nm, 20 °C] | +0,1061 |
| CCP-5OCF₂.F.F | 16,0 % | Δε [1 kHz, 20 °C] | |
| CUP-2F.F | 5,0 % | V_{(10,0,20)} [V] | 1,24 |
| CUP-3F.F | 5,0 % | V_{(90,0,20)} [V] | 1,92 |
| CUP-5F.F | 5,0 % | | |
| CUP-3OCCF₂.F.F | 10,0 % | | |
| CUP-5OCCF₂.F.F | 10,0 % | | |
| CBC-33F | 4,0 % | | |
| CBC-53F | 4,0 % | | |
| CBC-55F | 4,0 % | | |

| Beispiel 53 | | | |
|---|---|---|---|
| PCH-5F | 5,0 % | S → N [°C] | |
| CCP-2OCF₂.F.F | 16,0 % | Klärpunkt [°C] | +100 |
| CCP-3OCF₂.F.F | 15,0 % | Δn [589 nm, 20 °C] | +0,1077 |
| CCP-5OCF₂.F.F | 16,0 % | Δε [1 kHz, 20 °C] | |
| CUP-2F.F | 5,0 % | V_{(10,0,20)} [V] | 1,25 |
| CUP-3F.F | 5,0 % | V_{(90,0,20)} [V] | 1,97 |
| CUP-5F.F | 5,0 % | | |
| CUP-3OCCF₂.F.F | 10,0 % | | |
| CUP-5OCCF₂.F.F | 10,0 % | | |
| CBC-33F | 5,0 % | | |
| CBC-53F | 4,5 % | | |
| CBC-55F | 3,5 % | | |

| Beispiel 54 | | | |
|---|---|---|---|
| PCH-5F | 5,0 % | S → N [°C] | |
| PCH-7F | 6,0 % | Klärpunkt [°C] | +85 |
| CCP-2OCF₃ | 11,0 % | Δn [589 nm, 20 °C] | +0,0922 |
| CCP-3OCF₃ | 12,0 % | Δε [1 kHz, 20 °C] | |
| CCP-4OCF₃ | 10,0 % | V_{(10,0,20)} [V] | 1,41 |
| CCP-5OCF₃ | 12,0 % | V_{(90,0,20)} [V] | 2,20 |
| CUP-3OCH=CF₂.F.F | 12,0 % | | |
| CUP-5OCH=CF₂.F.F | 11,0 % | | |
| CCP-3F.F.F | 12,0 % | | |
| CCP-5F.F.F | 9,0 % | | |

| Beispiel 55 | | | |
|---|---|---|---|
| PCH-5F | 3,0 % | S → N [°C] | |
| CCP-2OCF₂.F.F | 16,0 % | Klärpunkt [°C] | +106 |
| CCP-3OCF₂.F.F | 15,0 % | Δn [589 nm, 20 °C] | +0,1117 |
| CCP-5OCF₂.F.F | 16,0 % | Δε [1 kHz, 20 °C] | |
| CUP-2F.F | 5,0 % | V_{(10,0,20)} [V] | 1,24 |
| CUP-3F.F | 5,0 % | V_{(90,0,20)} [V] | 1,94 |
| CUP-5F.F | 5,0 % | | |
| CUP-3OCH=CF₂.F.F | 10,0 % | | |
| CUP-5OCH=CF₂.F.F | 10,0 % | | |
| CBC-33F | 5,0 % | | |
| CBC-53F | 5,0 % | | |
| CBC-55F | 5,0 % | | |

| Beispiel 56 | | | |
|---|---|---|---|
| PCH-5F | 4,5 % | Klärpunkt [°C] | +99 |
| CCP-2OCF₂.F.F | 16,0 % | Δn [589 nm, 20 °C] | +0,1091 |
| CCP-3OCF₂.F.F | 15,0 % | V_{(10,0,20)} [V] | 1,12 |
| CCP-5OCF₂.F.F | 16,0 % | V_{(90,0,20)} [V] | 1,81 |
| CUP-2F.F | 5,0 % | | |
| CUP-3F.F | 5,0 % | | |
| CUP-5F.F | 5,0 % | | |
| CUP-3OCH=CF₂.F.F | 10,0 % | | |
| CUP-5OCH=CF₂.F.F | 10,0 % | | |
| CBC-33F | 5,0 % | | |
| CBC-53F | 4,5 % | | |
| CBC-55F | 4,0 % | | |

| Beispiel 57 | | | |
|---|---|---|---|
| PCH-5F | 4,0 % | Klärpunkt [°C] | +101 |
| CCP-2OCF₂.F.F | 16,0 % | Δn [589 nm, 20 °C] | +0,1098 |
| CCP-3OCF₂.F.F | 15,0 % | V_{(10,0,20)} [V] | 1,04 |
| CCP-5OCF₂.F.F | 16,0 % | V_{(90,0,20)} [V] | 1,68 |
| CUP-2F.F | 5,0 % | | |
| CUP-3F.F | 5,0 % | | |
| CUP-5F.F | 5,0 % | | |
| CUP-3OCH=CF₂.F.F | 10,0 % | | |
| CUP-5OCH=CF₂.F.F | 10,0 % | | |
| CBC-33F | 5,0 % | | |
| CBC-53F | 5,0 % | | |
| CBC-55F | 4,0 % | | |

| Beispiel 58 | | | |
|---|---|---|---|
| PCH-6F | 4,0 % | Klärpunkt [°C] | +85 |
| PCH-7F | 3,0 % | Δn [589 nm, 20 °C] | +0,1030 |
| CCP-2OCF₂.F.F | 16,0 % | V_{(10,0,20)} [V] | 1,15 |
| CCP-3OCF₂.F.F | 15,0 % | V_{(90,0,20)} [V] | 1,79 |
| CCP-5OCF₂.F.F | 17,0 % | | |
| CUP-2F.F | 6,0 % | | |
| CUP-3F.F | 6,0 % | | |
| CUP-5F.F | 5,0 % | | |
| CUP-3OCH=CF₂.F.F | 9,0 % | | |
| CUP-5OCH=CF₂.F.F | 9,0 % | | |
| CBC-33F | 4,0 % | | |
| CBC-53F | 3,0 % | | |
| CBC-55F | 3,0 % | | |

| Beispiel 59 | | | |
|---|---|---|---|
| PCH-6F | 3,0 % | Klärpunkt [°C] | +87 |
| CCP-2OCF₂.F.F | 16,0 % | Δn [589 nm, 20 °C] | +0,1071 |
| CCP-3OCF₂.F.F | 15,0 % | V_{(10,0,20)} [V] | 1,11 |
| CCP-5OCF₂.F.F | 16,0 % | V_{(90,0,20)} [V] | 1,74 |
| CUP-2F.F | 7,0 % | | |
| CUP-3F.F | 7,0 % | | |
| CUP-5F.F | 7,0 % | | |
| CUP-3OCH=CF₂.F.F | 10,0 % | | |
| CUP-5OCH=CF₂.F.F | 10,0 % | | |
| CBC-33F | 3,0 % | | |
| CBC-53F | 3,0 % | | |
| CBC-55F | 3,0 % | | |

| Beispiel 60 | | | |
|---|---|---|---|
| PCH-5F | 3,0 % | Klärpunkt [°C] | +84 |
| CCP-2OCF₂.F.F | 17,0 % | Δn [589 nm, 20 °C] | +0,1061 |
| CCP-3OCF₂.F.F | 15,0 % | V_{(10,0,20)} [V] | 1,10 |
| CCP-5OCF₂.F.F | 16,0 % | V_{(90,0,20)} [V] | 1,72 |
| CUP-2F.F | 7,0 % | | |
| CUP-3F.F | 7,0 % | | |
| CUP-5F.F | 7,0 % | | |
| CUP-3OCH=CF₂.F.F | 10,0 % | | |
| CUP-5OCH=CF₂.F.F | 10,0 % | | |
| CBC-33F | 3,0 % | | |
| CBC-53F | 3,0 % | | |
| CBC-55F | 2,0 % | | |

| Beispiel 61 | | | |
|---|---|---|---|
| PCH-5F | 4,0 % | Klärpunkt [°C] | +101 |
| CCP-2OCF₂.F.F | 16,0 % | Δn [589 nm, 20 °C] | +0,1105 |
| CCP-3OCF₂.F.F | 15,0 % | V_{(10,0,20)} [V] | 1,24 |
| CCP-5OCF₂.F.F | 16,0 % | V_{(90,0,20)} [V] | 1,93 |
| CUP-2F.F | 5,0 % | | |
| CUP-3F.F | 5,0 % | | |
| CUP-5F.F | 5,0 % | | |
| CUP-3OCH=CF₂.F.F | 10,0 % | | |
| CUP-5OCH=CF₂.F.F | 10,0 % | | |
| CBC-33F | 5,0 % | | |
| CBC-53F | 5,0 % | | |
| CBC-55F | 4,0 % | | |

| Beispiel 62 | | | |
|---|---|---|---|
| PCH-5F | 9,0 % | Klärpunkt [°C] | 84,6 |
| PCH-6F | 7,2 % | Δn [589 nm, 20 °C] | 0,0996 |
| PCH-7F | 5,4 % | Δε [1 kHz, 20 °C] | 6,37 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-2OCH=CF₂.F.F | 10,0 % | | |

| Beispiel 63 | | | |
|---|---|---|---|
| PCH-5F | 9,0 % | Klärpunkt [°C] | 87,3 |
| PCH-6F | 7,2 % | Δn [589 nm, 20 °C] | 0,0995 |
| PCH-7F | 5,4 % | Δε [1 kHz, 20 °C] | 6,22 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-5OCH=CF₂.F.F | 10,0 % | | |

| Beispiel 64 | | | |
|---|---|---|---|
| PCH-5F | 9,0 % | Klärpunkt [°C] | 86,6 |
| PCH-6F | 7,2 % | Δn [589 nm, 20 °C] | 0,0994 |
| PCH-7F | 5,4 % | Δε [1 kHz, 20 °C] | 6,48 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-3OCH=CF₂.F.F | 10,0 % | | |

| Beispiel 65 | | | |
|---|---|---|---|
| PCH-5F | 9,0 % | Klärpunkt [°C] | 87,6 |
| PCH-6F | 7,2 % | Δn [589 nm, 20 °C] | +0,0985 |
| PCH-7F | 5,4 % | Δε [1 kHz, 20 °C] | 6,61 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-3OC₂F₅.F.F | 10,0 % | | |

| Beispiel 66 | | | |
|---|---|---|---|
| PCH-5F | 9,0 % | Klärpunkt [°C] | 89 |
| PCH-6F | 7,2 % | Δn [589 nm, 20 °C] | +0,0985 |
| PCH-7F | 5,4 % | Δε [1 kHz, 20 °C] | 6,55 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-5OC₂F₅.F.F | 10,0 % | | |

| Beispiel 67 | | | |
|---|---|---|---|
| PCH-5F | 9,0 % | Klärpunkt [°C] | 90,3 |
| PCH-6F | 7,2 % | Δn [589 nm, 20 °C] | +0,0993 |
| PCH-7F | 5,4 % | Δε [1 kHz, 20 °C] | 6,22 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-5OC₂F₅.F | 10,0 % | | |

| Beispiel 68 | | | |
|---|---|---|---|
| PCH-5F | 9,0 % | Klärpunkt [°C]: | 89,3 |
| PCH-6F | 7,2 % | Δn [589 nm, 20 °C] | +0,0993 |
| PCH-7F | 5,4 % | Δε [1 kHz, 20 °C] | 6,27 |
| CCP-2OCF₃ | 7,2 % | | |
| CCP-3OCF₃ | 10,8 % | | |
| CCP-4OCF₃ | 8,1 % | | |
| CCP-5OCF₃ | 8,1 % | | |
| BCH-3F.F | 10,8 % | | |
| BCH-5F.F | 9,0 % | | |
| ECCP-3OCF₃ | 4,5 % | | |
| ECCP-5OCF₃ | 4,5 % | | |
| CBC-33F | 1,8 % | | |
| CBC-53F | 1,8 % | | |
| CBC-55F | 1,8 % | | |
| CUP-3OC₂F₅.F | 10,0 % | | |

## Patentansprüche

1. Flüssigkristallines Medium auf der Basis eines Gemisches von polaren Verbindungen mit positiver dielektrischer Anisotropie, dadurch gekennzeichnet, daß es eine oder mehrere Verbindungen der allgemeinen Formel I 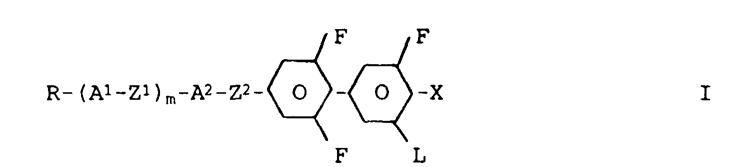 enthält,
zusätzlich eine oder mehrere Verbindungen ausgewählt aus der Gruppe bestehend aus den allgemeinen Formeln II, III, IV und V enthält: 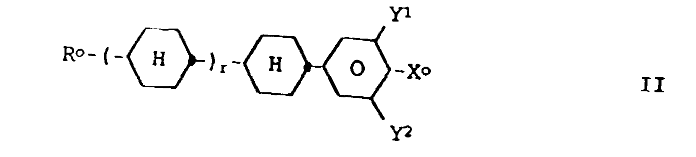 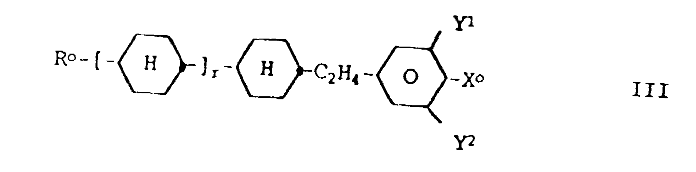 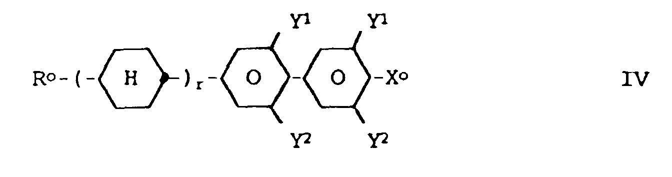 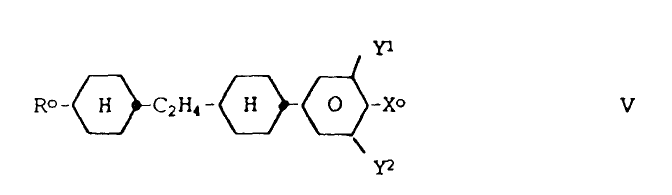 worin die einzelnen Reste die folgenden Bedeutungen haben:
R H, einen unsubstituierten, einen einfach durch CN oder CF₃ oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, -<>-, -CO-, -CO-O-, -O-CO- oder -O-CO-O- so ersetzt sein können, daß O-Atome nicht direkt miteinander verknüpft sind,
A¹ und A² jeweils unabhängig voneinander
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch ein oder zwei Fluor substituiert sein können,
Z¹ und Z² jeweils unabhängig voneinander -CO-O-, -O- CO-, -CH₂O-, -OCH₂-, -CH₂CH₂-, -CH=CH-, -C≡C- oder eine Einfachbindung, einer der Reste Z¹ und Z² auch -(CH₂)₄- oder -CH=CH-CH₂CH₂-,
X halogeniertes Alkyl, Alkoxy, Alkenyl oder Alkenyloxy mit jeweils 1 bis 6 C-Atomen,
L F und im Fall X = OCF₂H oder OC₂F₅ auch H, und
m 0, 1 oder 2,
R^{o}: Alkyl, Oxaalkyl, Fluoralkyl oder Alkenyl mit jeweils bis zu 7 C-Atomen,
X^{o} : F, Cl, CF₃, OCF₃, OCHF₂, OCH=CF₂, OCF=CF₂, OCFH=CF₂H oder OCF₂-CF₂H,
Y¹ und Y²: jeweils unabhängig voneinander H oder F,
r: 0 oder 1.

2. Medium nach Anspruch 1 , dadurch gekennzeichnet, daß es zusätzlich eine oder mehrere Verbindungen ausgewählt aus der Gruppe bestehend aus den allgemeinen Formeln VI bis XII enthält: 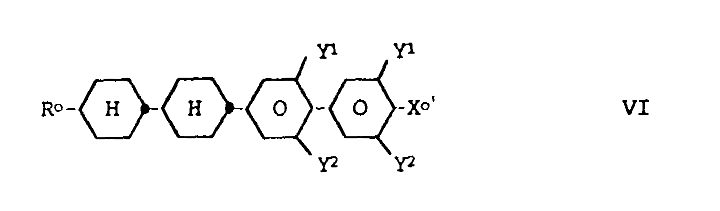 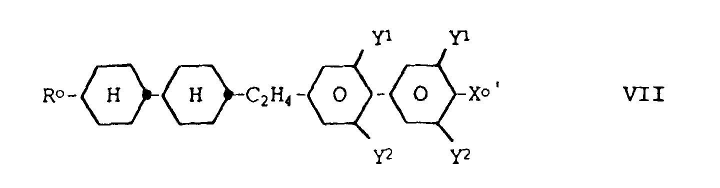 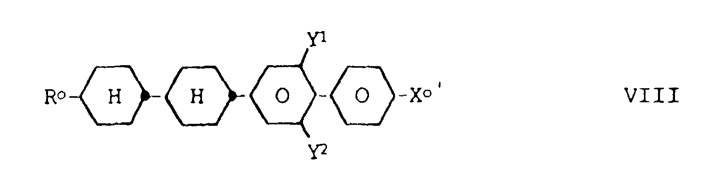 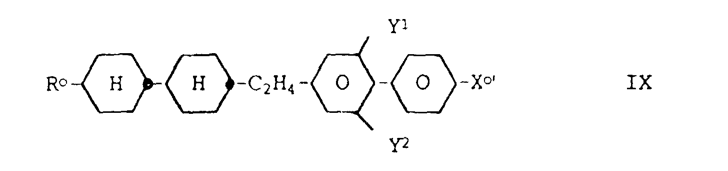 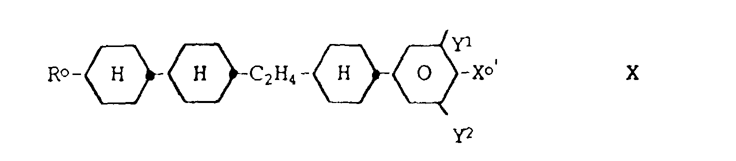 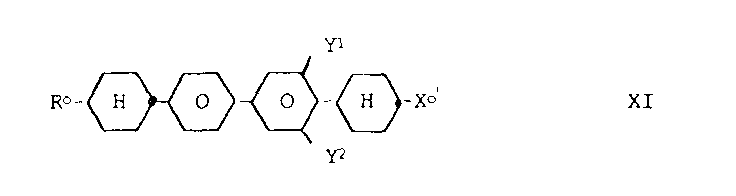 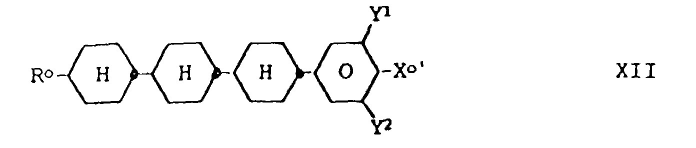 wenn R^{o}, Y¹ und Y² jeweils unabhängig voneinander eine der in Anspruch 1 angegebenen Bedeutung haben und
x°^{'} F, Cl, CF₃, OCF₃, OCHF₂, Alkyl, Oxaalkyl, Fluoralkyl oder Alkenyl mit jeweils 7 C-Atomen bedeutet.

3. Medium nach Anspruch¹ , dadurch gekennzeichnet, daß der Anteil an Verbindungen der Formeln I bis V zusammen im Gesamtgemisch mindestens 50 Gew.-% beträgt.

4. Medium nach Anspruch 1 , dadurch gekennzeichnet, daß der Anteil an Verbindungen der Formel I im Gesamtgemisch 3 bis 80 Gew.-% beträgt.

5. Medium nach mindestens einem der Ansprüche 1 bis 3, dadurch gekennzeichnet, daß der Anteil an Verbindungen der Formeln II bis V im Gesamtgemisch 20 bis 80 Gew.-% beträgt.

6. Medium nach Anspruch 1 oder 2, dadurch gekennzeichnet, daß es im wesentlichen aus Verbindungen ausgewählt aus der Gruppe bestehend aus den allgemeinen Formeln I bis XII besteht.

7. Verwendung des flüssigkristallinen Mediums nach Anspruch 1 für elektrooptische Zwecke.

8. Medium nach mindestens einem der Ansprüche 1 bis 6, dadurch gekennzeichnet, daß es eine oder mehrere Verbindungen der Formel 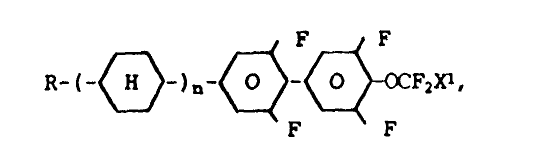 worin
R die in Anspruch 1 angegebene Bedeutung haben, n 1 oder 2 und X¹ H, F oder Cl bedeutet,
enthält.

9. Medium nach mindestens einem der Ansprüche 1 bis 6, dadurch gekennzeichnet, daß es eine oder mehrere Verbindungen der Formel 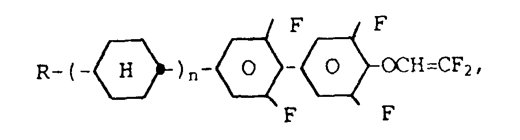 worin
R die in Anspruch 1 angegebene Bedeutung hat und n 1 oder 2 ist,
enthält.

10. Medium nach mindestens einem der Ansprüche 1 bis 6 , dadurch gekennzeichnet, daß es eine oder mehrere Verbindungen der Formel 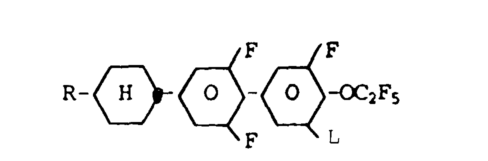 worin R die in Anspruch 1 angegebene Bedeutung hat und L H oder F bedeutet.

11. Medium nach mindestens einem der Ansprüche 1 bis 6, dadurch gekennzeichnet, daß es eine oder mehrere Verbindungen der Formel, 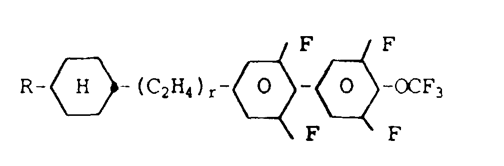 worin
R die in Anspruch 1 angegebene Bedeutung hat und r 0 oder 1 ist.
enthält.

12. Elektrooptische Flüssigkristallanzeige enthaltend ein flüssigkristallines Medium nach Anspruch 1.

13. Tetrafluorbiphenyle der Formel II, 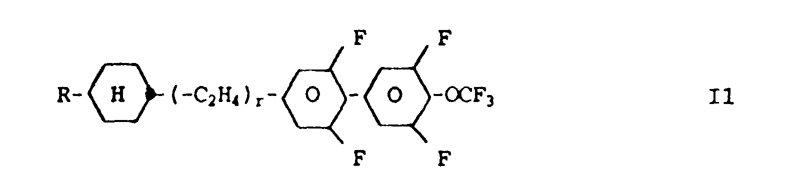 worin
R die in Anspruch 1 angegebene Bedeutung hat und r 0 oder 1 bedeutet.

## Claims

1. Liquid-crystalline medium based on a mixture of polar compounds having positive dielectric anisotropy, characterized in that it comprises one or more compounds of the general formula I 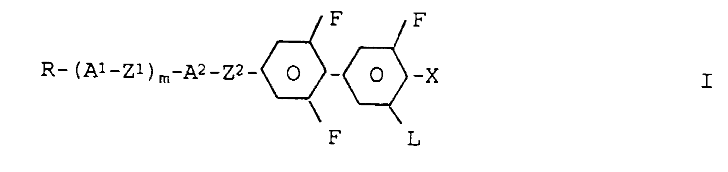 and additionally comprises one or more compounds selected from the group consisting of the general formulae II, III, IV and V: 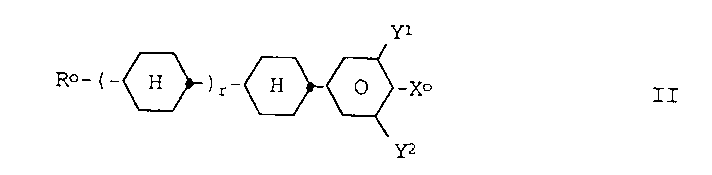 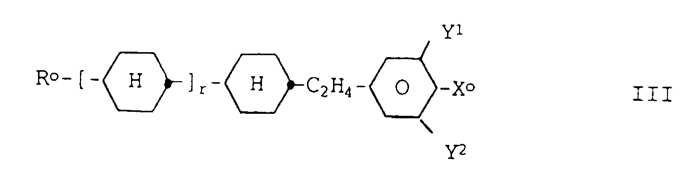 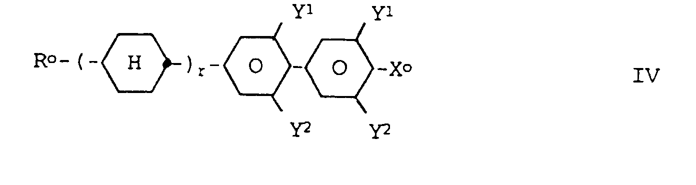 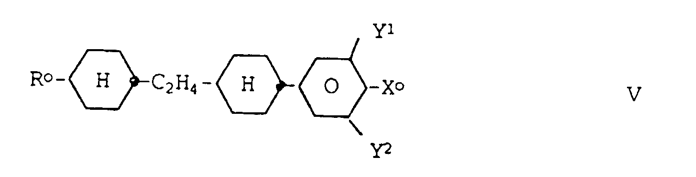 in which the individual radicals are as defined below:
R is H, an alkyl or alkenyl radical having 1 to 15 carbon atoms which is unsubstituted, monosubstituted by CN or CF₃ or at least monosubstituted by halogen, it also being possible for one or more CH₂ groups in these radicals to be replaced, in each case independently of one another, by -O-, -S-, -<>-, -CO-, -CO-O-, -O-CO- or -O-CO-O-, in such a way that O atoms are not linked directly to one another,
A¹ and A² are each, independently of one another, a
(a) trans-1,4-cyclohexylene radical in which, in addition, one or more non-adjacent CH₂ groups may be replaced by -O- and/or -S-,
(b) 1,4-phenylene radical in which, in addition, one or two CH groups may be replaced by N, or
(c) radical from the group consisting of 1,4-cyclohexenylene, 1,4-bicyclo(2,2,2)-octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by one or two fluorine atoms,
Z¹ and Z² are each, independently of one another, -CO-O-, -O-CO-, -CH₂O-, -OCH₂-, -CH₂CH₂-, -CH=CH-, -C≡C- or a single bond, and one of the radicals z¹ and Z² is alternatively -(CH₂)₄- or -CH=CH-CH₂CH₂-,
X is halogenated alkyl, alkoxy, alkenyl or alkenyloxy, in each case having 1 to 6 carbon atoms,
L is F and also H when X is OCF₂H or OC₂F₅, and
m is 0, 1 or 2,
R^{o}: alkyl, oxaalkyl, fluoroalkyl or alkenyl, in each case having up to 7 carbon atoms,
X⁰: F, Cl, CF₃, OCF₃, OCHF₂, OCH=CF₂, OCF=CF₂, OCFH-CF₂H or OCF₂-CF₂H,
Y¹ and Y²: each, independently of one another, H or F
r: 0 or 1.

2. Medium according to Claim 1, characterized in that it additionally comprises one or more compounds selected from the group consisting of the general formulae VI to XII: 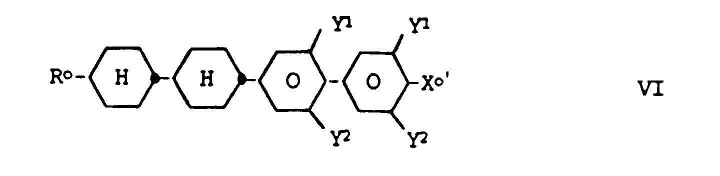 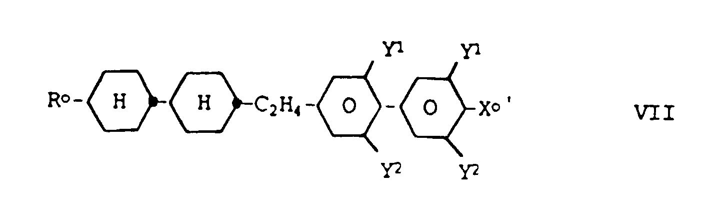 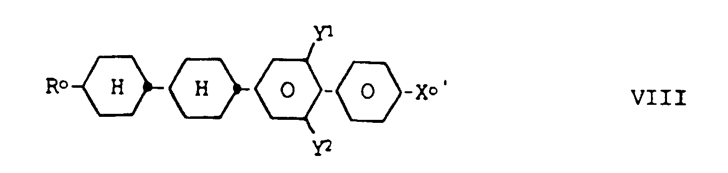 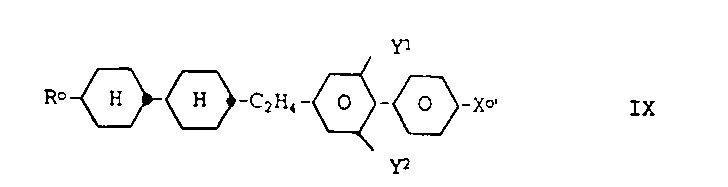 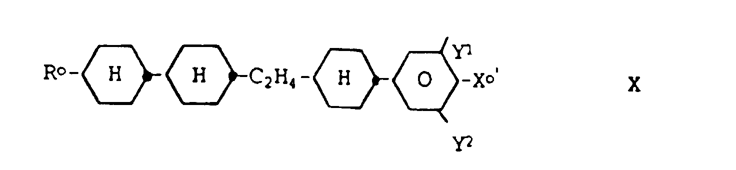 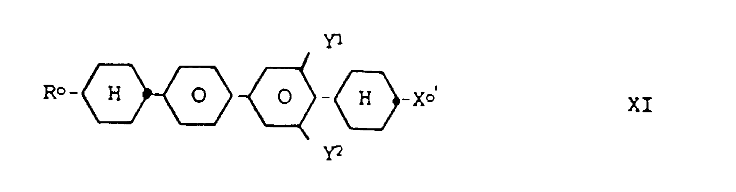 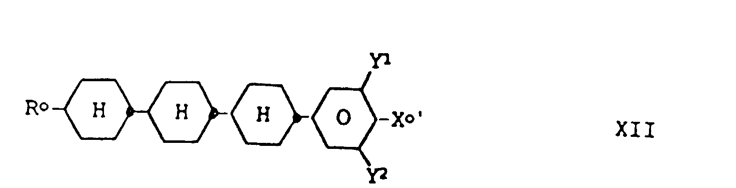 if
R^{o}, Y¹ and Y² each, independently of one another, have one of the meanings given in Claim 1, and
X^{o}' is F, Cl, CF₃, OCF₃, OCHF₂, alkyl, oxaalkyl, fluoroalkyl or alkenyl, in each case having 7 carbon atoms.

3. Medium according to Claim 1, characterized in that the proportion of compounds of the formulae I to V together is at least 50% by weight in the total mixture.

4. Medium according to Claim 1, characterized in that the proportion of compounds of the formula I is from 3 to 80% by weight in the total mixture.

5. Medium according to at least one of Claims 1 to 3, characterized in that the proportion of compounds of the formulae II to V is from 20 to 80% by weight in the total mixture.

6. Medium according to Claim 1 or 2, characterized in that it essentially consists of compounds selected from the group consisting of the general formulae I to XII.

7. Use of the liquid-crystalline medium according to Claim 1 for electrooptical purposes.

8. Medium according to at least one of Claims 1 to 6, characterized in that it comprises one or more compounds of the formula 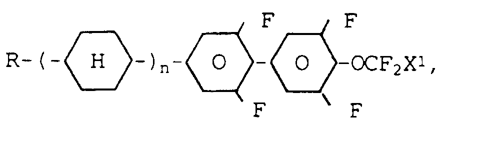 in which
R is as defined in Claim 1, n is 1 or 2 and X' is H, F or Cl.

9. Medium according to at least one of Claims 1 to 6, characterized in that it comprises one or more compounds of the formula 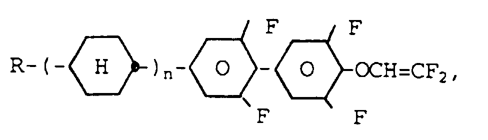 in which
R is as defined in Claim 1 and n is 1 or 2.

10. Medium according to at least one of Claims 1 to 6, characterized in that it comprises one or more compounds of the formula 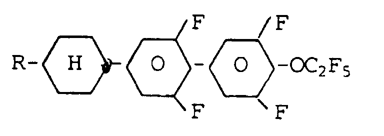 in which
R is as defined in Claim 1 and L is H or F.

11. Medium according to at least one of Claims 1 to 6, characterized in that it comprises one or more compounds of the formula 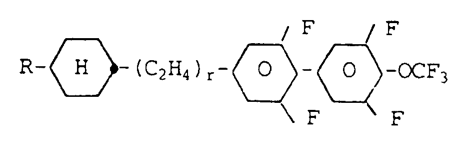 in which
R is as defined in Claim 1 and r is O or 1.

12. Electrooptical liquid-crystal display containing a liquid-crystalline medium according to Claim 1.

13. Tetrafluorobiphenyls of the formula I1 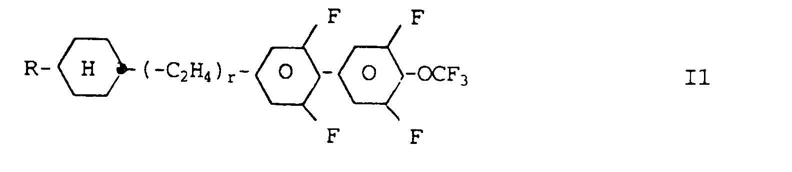 in which
R is as defined in Claim 1 and r is O or 1.

## Revendications

1. Milieu à cristaux liquides à base d'un mélange de composés polaires à anisotropie diélectrique, caractérisé en ce qu'il contient un ou plusieurs composés de formule générale I 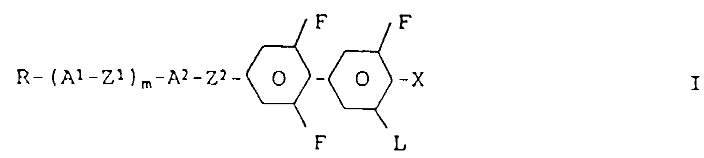 et en outre un ou plusieurs composés choisis dans l'ensemble constitué par ceux de formules générales II, III, IV et V: 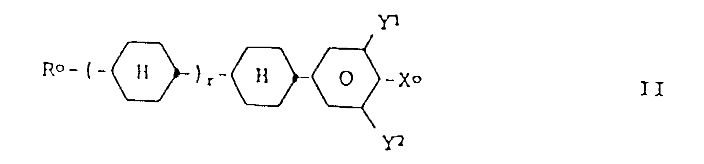 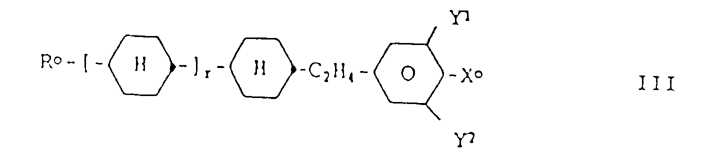 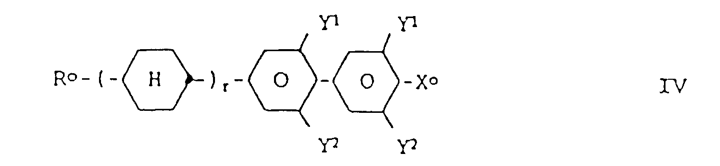 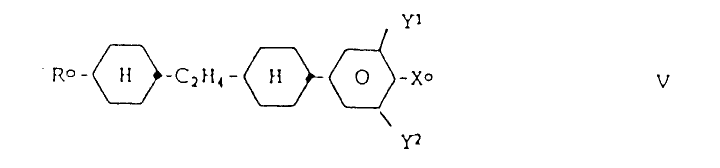 dans lesquelles les radicaux individuels ont les significations suivantes:
R représente H ou un radical alkyle ou alcényle ayant de 1 à 15 atomes de carbone, non substitué ou substitué une seule fois par CN ou CF₃ ou substitué au moins une fois par un atome d'halogène, dans ces radicaux un ou plusieurs groupes CH₂ pouvant également être remplacés chacun, indépendamment les uns des autres, par -O-, -S-, -<>-, -CO-, -CO-O-, -O-CO- ou -O-CO-O-, de manière que des atomes d'oxygène ne soient pas directement liés les uns aux autres,
A¹ et A² représentent chacun, indépendamment l'un de l'autre,
(a) un radical *trans*-1,4-cyclohexylène*,* dans lequel un ou plusieurs groupes CH₂ non contigus peuvent également être remplacés par -O- et/ou -S-,
(b) un radical 1,4-phénylène, dans lequel un ou deux groupes CH peuvent également être remplacés par N,
(c) un radical choisi parmi les groupes 1,4-cyclohexénylène, 1,4-bicyclo(2,2,2)-octylène, pipéridine-1,4-diyle, naphtalène-2,6-diyle, décahydronaphtalène-2,6-diyle et 1,2,3,4-tétrahydronaphtalène-2,6-diyle,
les radicaux (a) et (b) pouvant être substitués par un ou deux atomes de fluor,
Z¹ et Z² représentent chacun, indépendamment l'un de l'autre, -CO-O-, -O-CO-, -CH₂O-, -OCH₂-, -CH₂CH₂-, -CH=CH-, -C≡C- ou une liaison simple, l'un des radicaux Z¹ et Z² pouvant également représenter -(CH₂)₄- ou -CH=CH-CH₂CH₂-,
X représente un groupe alkyle, alcoxy, alcényle ou alcényloxy, halogéné, ayant dans chaque cas de 1 à 6 atome de carbone,
L représente un atome de fluor, ou, lorsque X = OCF₂H ou OC₂F₅, également un atome d'hydrogène, et
m représente 0, 1 ou 2;
R^{o} représente un groupe alkyle, oxaalkyle, fluoroalkyle ou alcényle ayant chacun jusqu'à 7 atomes de carbone,
X^{o} représente F, Cl, CF₃, OCF₃, OCHF₂, OCH=CF₂, OCF=CF₂, OCFH-CF₂H ou OCF₂-CF₂H,
Y¹ et Y² représentent chacun, indépendamment l'un de l'autre, H ou F,
r est 0 ou 1.

2. Milieu selon la revendication 1, caractérisé en ce qu'il contient en outre un ou plusieurs composés choisis dans l'ensemble constitué par les composés de formules générales VI à XII: 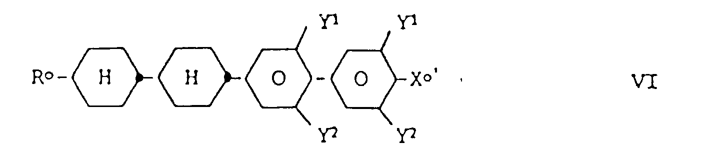 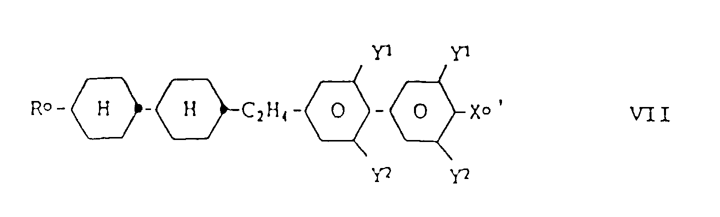 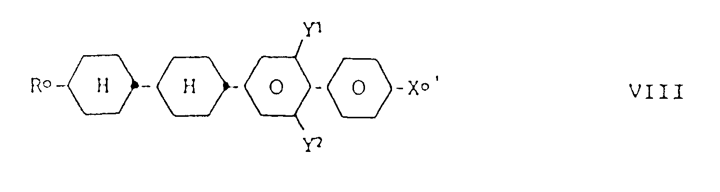 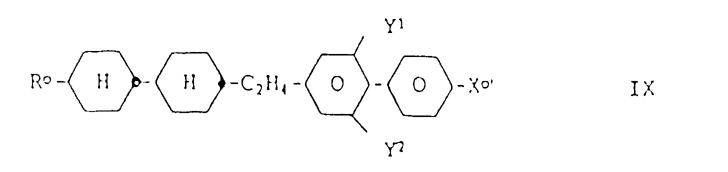 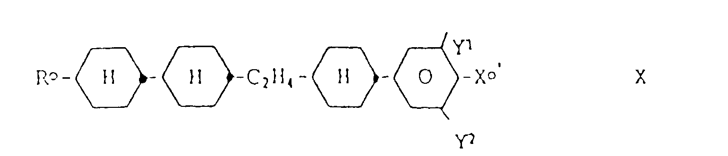 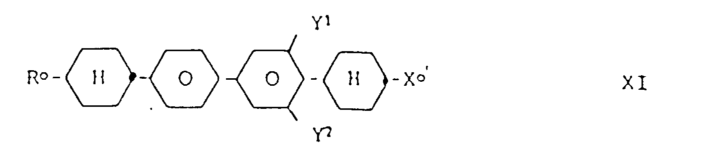 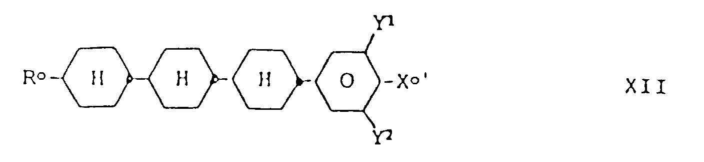 dans lesquelles R^{o}, Y¹ et Y² ont chacun, indépendamment les uns des autres, l'une des significations données dans la revendication 1, et
X°', représente un atome de fluor ou de chlore, ou le groupe CF₃, OCF₃, OCHF₂ ou un groupe alkyle, oxaalkyle, fluoroalkyle ou alcényle ayant dans chaque cas 7 atomes de carbone.

3. Milieu selon la revendication 1, caractérisé en ce que la proportion des composés de formules I à V ensemble dans le mélange total est d'au moins 50 % en poids.

4. Milieu selon la revendication 1, caractérisé en ce que la proportion des composés de formules I dans le mélange total va de 3 à 80 % en poids.

5. Milieu selon au moins une des revendications 1 à 3, caractérisé en ce que la proportion des composés de formules II à V ensemble dans le mélange total va de 20 à 80 % en poids.

6. Milieu selon la revendication 1 ou 2, caractérisé en ce qu'il consiste essentiellement en composés choisis dans l'ensemble constitué par les composés de formules générales I à XII.

7. Utilisation du milieu à cristaux liquides selon la revendication 1, à des fins électro-optiques.

8. Milieu selon au moins une des revendications 1 à 6, caractérisé en ce qu'il contient un ou plusieurs composés de formule 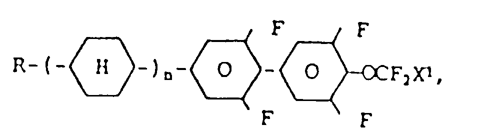 dans laquelle
R a la signification donnée dans la revendication 1,
n est 1 ou 2 et
X¹ représente H, F ou Cl.

9. Milieu selon au moins une des revendications 1 à 6, caractérisé en ce qu'il contient un ou plusieurs composés de formule 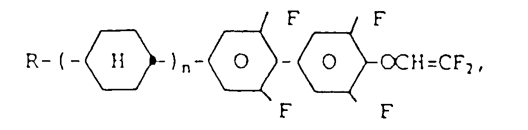 dans laquelle
R a la signification donnée dans la revendication 1 et n est 1 ou 2.

10. Milieu selon au moins une des revendications 1 à 6, caractérisé en ce qu'il contient un ou plusieurs composés de formule 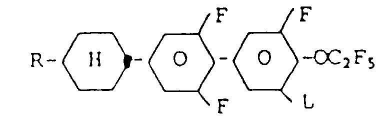 dans laquelle
R a la signification donnée dans la revendication 1 et
L représente H ou F.

11. Milieu selon au moins une des revendications 1 à 6, caractérisé en ce qu'il contient un ou plusieurs composés de formule 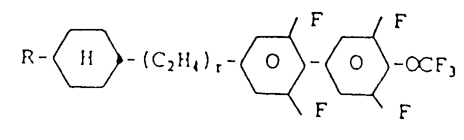 dans laquelle
R a la signification donnée dans la revendication 1 et
r est 0 ou 1.

12. Afficheur électro-optique à cristaux liquides, contenant un milieu à cristaux liquides selon la revendication 1.

13. Tétrafluorobiphényles de formule I1 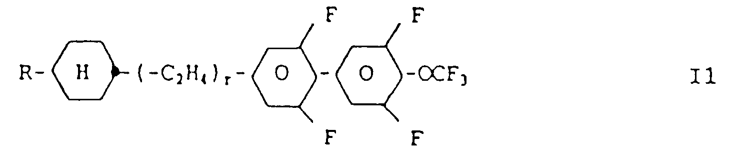 dans laquelle
R a la signification donnée dans la revendication 1 et
r est 0 ou 1.
